(19) Europäisches Patentamt
European Patent Office
Office européen des brevets

(11) Veröffentlichungsnummer: **0 574 846 A2**

# (12) EUROPÄISCHE PATENTANMELDUNG

(21) Anmeldenummer: **93109410.6**

(22) Anmeldetag: **11.06.93**

(51) Int. Cl.5: **C07D 471/04**, A61K 31/435, //(C07D471/04,235:00,221:00)

(30) Priorität: **17.06.92 DE 4219818**
**24.02.93 DE 4305602**

(43) Veröffentlichungstag der Anmeldung:
**22.12.93 Patentblatt 93/51**

(84) Benannte Vertragsstaaten:
**AT BE CH DE DK ES FR GB GR IE IT LI LU NL PT SE**

(71) Anmelder: **MERCK PATENT GmbH**
**Postfach,**
**Frankfurter Strasse 250**
**D-64271 Darmstadt(DE)**

(72) Erfinder: **Mederski, Werner, Dr.**
**Am Ohlenberg 29**
**D-64390 Erzhausen(DE)**
Erfinder: **Dorsch, Dieter, Dr.**
**Königsberg Strasse 17a**
**D-64372 Ober-Ramstadt(DE)**
Erfinder: **Osswald, Mathias, Dr.**
**Katzenelnbogenweg 1**
**D-64673 Zwingenberg(DE)**
Erfinder: **Beier, Norbert, Dr.**
**Georgenhäuser Strasse 19**
**D-64354 Reinheim(DE)**
Erfinder: **Schelling, Pierre, Prof. Dr.**
**Bordenbergweg 17**
**D-64367 Mühltal(DE)**
Erfinder: **Lues, Ingeborg, Dr.**
**Katharinenstrasse 2**
**D-64297 Darmstadt(DE)**
Erfinder: **Minck, Klaus-Otto, Dr.**
**Büchestrasse 8**
**W-64372 Ober-Ramstadt(DE)**

## (54) Imidazopyridine als Angiotensin II Antigonisten.

(57) Neue Imidazopyridinderivate der Formel I

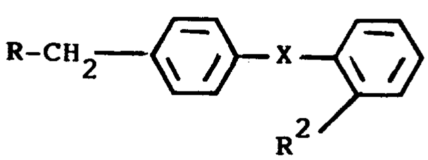

I

worin
R

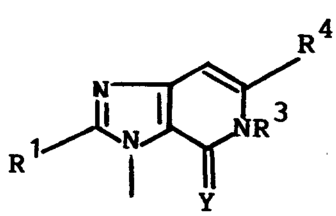

bedeutet und $R^1$, $R^2$, $R^3$, $R^4$, X und Y die in Patentanspruch 1 angegebenen Bedeutungen haben, sowie deren Salze zeigen Angiotensin II-antagonistische Eigenschaften und können zur Behandlung von Hypertension,

EP 0 574 846 A2

Aldosteronismus, Herzinsuffizienz und erhöhtem Augeninnendruck sowie von Störungen des Zentralnervensystems verwendet werden.

Die Erfindung betrifft neue Imidazopyridinderivate der Formel I

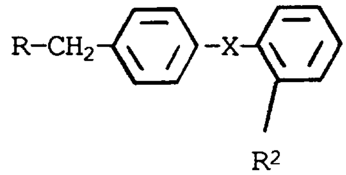

I

worin

R

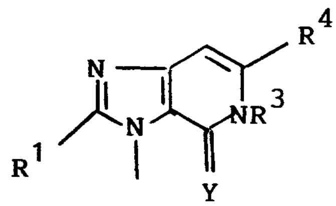

| | |
|---|---|
| $R^1$ | A, Alkenyl oder Alkinyl mit jeweils bis zu 6 C-Atomen, $C_3$-$C_7$-Cycloalkyl-$C_kH_{2k}$- oder $C_1$-$C_6$-Alkyl, worin eine $CH_2$-Gruppe durch O oder S ersetzt ist, |
| $R^2$ | H, COOH, COOA, CN, $NO_2$, $NHCOR^5$, $NHSO_2R^5$ oder 1H-5-Tetrazolyl, |
| $R^3$ | ein- oder mehrfach durch COOH, COOA, CN, $NO_2$, $NR^6R^7$, $NHCOR^8$, $NHSO_2R^8$, Hal und/oder Ar substituiertes Alkenyl mit 2-6 C-Atomen im "alkenyl"-Teil, $-C_nH_{2n}-R^9$ oder $-CHR^{10}-C_kH_{2k}-R^{11}$, |
| $R^4$ | H oder Hal, |
| $R^5$ und $R^8$ | jeweils Alkyl mit 1-5 C-Atomen, worin auch ein oder mehrere H-Atom(e) durch F ersetzt sein können, |
| $R^6$ und $R^7$ | jeweils H, A, Alkenyl oder Alkinyl mit jeweils bis zu 6 C-Atomen, Ar, $ArC_nH_{2n}$- oder $Het^2$, |
| $R^6$ auch | $-CH_2COOA$, $-SO_2$-A oder $-SO_2$-Ar, |
| $R^6$ und $R^7$ | zusammen auch eine Alkylenkette mit 2-5 C-Atomen, die ein- oder mehrfach durch Carbonylsauerstoff, Ar, $Het^2$, -CO-Ar, -COOA, $-CO-N(A)_2$, $-CH_2OH$, $-SO_2$-Ar und/oder -NH-CO-A substituiert und/oder durch O oder durch $-NR^{16}$- unterbrochen sein kann, |
| $R^9$ | Cycloalkyl mit 3-8 C-Atomen, $Het^1$, $-CO-NR^6R^7$, $-CO-R^{14}$, $-C(=NR^{12})$-A, $-C(=NR^{12})-Het^2$, $-S(O)_m$-A, $-S(O)_m$-Ar, $-S(O)_m-Het^2$, $-SO_2-NH-Het^2$ oder $-SO_2-OR^{15}$, |
| $R^{10}$ | COOH, COOA, CN, $NO_2$, $NHCOR^{11}$, $NHSO_2R^{11}$ oder 1H-5-Tetrazolyl, |
| $R^{11}$ | Ar oder Cycloalkyl mit 3-8 C-Atomen, |
| $R^{12}$ | H, OH, CN, $R^{13}$, $OR^{13}$ oder OAr, |
| $R^{13}$ | A, Alkenyl oder Alkinyl mit jeweils bis zu 6 C-Atomen, |
| $R^{14}$ | $-NH-CHR^{15}-COOH$, $-NH-CHR^{15}-COOA$, $-CH_2S(O)_m$-Ar, $-CH_2$-COOA, $-C_nH_{2n}-NO_2$, $-C_nH_{2n}-NR^6R^7$ oder $-C_nH_{2n}$-NHCOOA, |
| $R^{15}$ | H oder A, |
| $R^{16}$ | H, A, Ar, COOA, $Het^2$ oder $SO_2$-Ar, |
| X | fehlt, -NH-CO-, -CO-NH-, -O-CH(COOH)-, -NH-CH(COOH)-, -NA-CH(COOH), -CH=C-(COOH), -CH=C(CN) oder -CH=C(1H-5-tetrazolyl)-, |
| Y | O oder S, |
| A | Alkyl mit 1-6 C-Atomen, |
| Ar | eine unsubstituierte oder eine durch $R^5$, $OR^5$, COOH, COOA, CN, $NO_2$, $NH_2$, $NHCOR^5$, $NHSO_2R^5$, Hal oder 1H-5-Tetrazolyl mono- oder disubstituierte Phenylgruppe, |
| $Het^1$ | einen fünf- oder sechsgliedrigen gesättigten heterocyclischen Rest mit 1 bis 3 N-, O- und/oder S-Atomen, der einfach durch Carbonylsauerstoff oder $=NR^{12}$ und/oder dessen Ring-N-Atom(e) jeweils durch A oder Ar substituiert sein kann (können), |
| $Het^2$ | einen fünf- oder sechsgliedrigen heteroaromatischen Rest mit 1 bis 3 N-, O- und/oder S-Atomen, der auch mit einem Benzol- oder Pyridinring kondensiert sein kann und/oder ein- oder mehrfach durch A substituiert sein kann, |

Hal F, Cl, Br oder I,
k 0, 1, 2, 3 oder 4,
m 0, 1 oder 2 und
n 1, 2, 3, 4, 5 oder 6 bedeuten,

sowie ihre Salze.

Ähnliche Verbindungen sind aus der EP-A2-0400 974 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher als Arzneimittelwirkstoffe zur Prophylaxe und/oder Therapie von Herz-, Kreislauf- und Gefäßkrankheiten, vor allem zur Behandlung der angiotensin II-abhängigen Hypertension, des Aldosteronismus, der Herzinsuffizienz und des erhöhten Augeninnendrucks sowie von Störungen des Zentralnervensystems eingesetzt werden, ferner von Hypertrophie und Hyperplasie der Blutgefäße und des Herzens, Angina pectoris, Herzinfarkt, Schlaganfall, Restenosen nach Angioplastie oder By-pass-Operationen, Arteriosklerose, Glaukomen, macularer Degeneration, Hyperurikämie, Nierenfunktionsstörungen, z.B. Nierenversagen, Nephropathia diabetica, Retinopathia diabetica, Psoriasis, angiotensin II-vermittelten Störungen in weiblichen Fortpflanzungsorganen, Wahrnehmungsstörungen, z.B. Demenz, Amnesie, Gedächtnisfunktionsstörungen, Angstzuständen, Depression und/oder Epilepsie.

Diese Wirkungen können nach üblichen in vitro- oder in vivo-Methoden ermittelt werden, wie sie z.B. in der US-PS 4 880 804, der US-PS 5 036 048 und der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Gegenstand der Erfindung sind die Verbindungen der Formel I und ihre Salze sowie ein Verfahren zur Herstellung dieser Verbindungen sowie ihrer Salze, dadurch gekennzeichnet, daß man

(a) eine Verbindung der Formel II

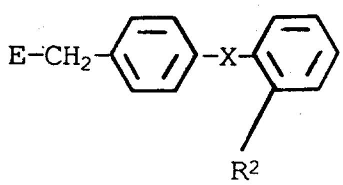

II

worin

E Cl, Br, I oder eine freie oder reaktionsfähig funktionell angewandelte OH-Gruppe bedeutet und

$R^2$ und X die in Anspruch 1 angegebene Bedeutung haben,

mit einer Verbindung der Formel III

H-R III

worin

R die in Anspruch 1 angegebene Bedeutung hat,

umsetzt

oder

(b) eine Verbindung der Formel IV

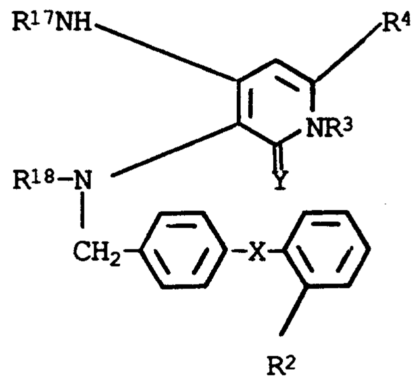

IV

worin

$R^{17}$ $R^1$-CO oder H und

$R^{18}$ H (falls $R^{17}$ $R^1$-CO ist) oder $R^1$-CO (falls $R^{17}$ H ist)

bedeuten

und

$R^1$, $R^2$, $R^3$, $R^4$, X und Y die in Anspruch 1 angegebenen Bedeutungen haben,

mit einem cyclisierenden Mittel behandelt,

oder

(c) zur Herstellung einer Verbindung der Formel I, worin X -NH-CO- oder -CO-NH- bedeutet, eine Verbindung der Formel V

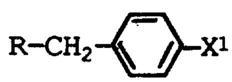

V

worin

$X^1$ $NH_2$ oder COOH bedeutet und

R die in Anspruch 1 angegebene Bedeutung hat

oder ein reaktionsfähiges Derivat dieser Verbindung mit einer Verbindung der Formel VI

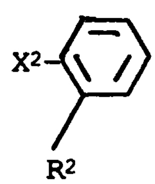

VI

worin

$X^2$ COOH (falls $X^1$ $NH_2$ ist) oder $NH_2$ (falls $X^1$ COOH ist) bedeutet und

$R^2$ die in Anspruch 1 angegebene Bedeutung hat,

oder mit einem reaktionsfähigen Derivat dieser Verbindung umsetzt oder

(d) eine Verbindung der Formel VII

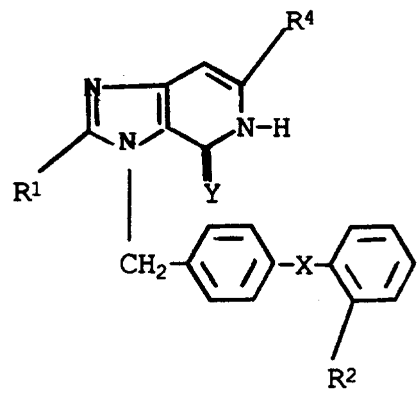

VII

worin

$R^1$, $R^2$, $R^4$, X und Y die in Anspruch 1 angegebenen Bedeutungen haben,

mit einer Verbindung der Formel VIII

E-$R^3$ VIII

worin

$R^3$ und E die in Anspruch 1 bzw. 3 angegebenen Bedeutungen haben,

umsetzt oder

(e) zur Herstellung einer Verbindung der Formel I, die eine -C(=N$R^{12}$)-Gruppe enthält, eine entsprechende Carbonylverbindung mit einer Verbindung der Formel $H_2N$-$R^{12}$, worin $R^{12}$ die in Anspruch 1 angegebene Bedeutung hat, behandelt oder

(f) zur Herstellung einer Verbindung, worin $R^3$ -$C_n$-$H_{2n}$-CO-$R^{19}$ und $R^{19}$-N$R^6R^7$ oder -NH-CH$R^{15}$-COOA bedeutet, eine Carbonsäure, die der Formel I entspricht, aber an Stelle des Restes $R^3$ eine -$C_n$-$H_{2n}$-COOH-Gruppe enthält (oder eines ihrer funktionellen Derivate) mit einer Verbindung der Formel H-$R^{19}$ umsetzt oder

(g) eine Verbindung der Formel I aus einem ihrer funktionellen Derivate durch Behandeln mit einem solvolysierenden oder hydrogenolysierenden Mittel in Freiheit setzt,

und/oder daß man in einer Verbindung der Formel I einen oder mehrere Rest(e) R und/oder $R^2$ in einen oder mehrere andere Reste R und/oder $R^2$ umwandelt und/oder eine Base oder Säure der Formel I in eines ihrer Salze umwandelt.

Vor- und nachstehend haben die Reste bzw. Parameter R, $R^1$ bis $R^{18}$, X, Y, A, Ar, $Het^1$, $Het^2$, Hal,k,m,n, E, $X^1$ und $X^2$ die bei den Formeln I bis VIII angegebenen Bedeutungen, falls nicht ausdrücklich etwas anderes angegeben ist.

In den vorstehenden Formeln hat A 1-6, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl. 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Alkenyl steht vorzugsweise für Vinyl, 1- oder 2-Propenyl, 1-Butenyl, ferner 1-Pentenyl oder 1-Hexenyl. Alkinyl steht vorzugsweise für Ethinyl, 1- oder 2-Propinyl, ferner 1-Butinyl, 1-Pentinyl oder 1-Hexinyl. Falls mehrere Reste A, Alkenyl oder Alkinyl in einer Verbindung der Formel I vorhanden sind, so können sie gleich oder voneinander verschieden sein.

Hal bedeutet vorzugsweise F, Cl oder Br, aber auch J.

R ist ein von 3H-Imidazo[4,5-c]pyridin ("3H-IP") abgeleiteter Rest, genauer 2-$R^1$-4-(thi)oxo-5-$R^3$-6-$R^4$-4,5-dihydro-3H-imidazo[4,5-c]pyridin-3-yl.

Ar ist vorzugsweise unsubstituiertes, ferner - wie angegeben - monosubstituiertes Phenyl, im einzelnen bevorzugt Phenyl, o-, m- oder p-Tolyl, o-, m- oder p-Ethylphenyl, o-, m- oder p-Trifluormethylphenyl, o-, m- oder p-Methoxyphenyl, o-, m-oder p-Ethoxyphenyl, o-, m- oder p-(Difluormethoxy)-phenyl, o-, m- oder p-Trifluormethoxyphenyl, o-, m- oder p-Carboxyphenyl, o-, m- oder p-Methoxycarbonylphenyl, o-, m- oder p-Ethoxycarbonylphenyl, o-, m- oder p-Cyanphenyl, o-, m- oder p-Nitrophenyl, o-, m- oder p-Aminophenyl, o-, m- oder p-Acetamidophenyl, o-, m- oder p-Trifluoracetamidophenyl, o-, m- oder p-Methylsulfonamidophenyl, o-, m- oder p-Trifluormethylsulfonamidophenyl, o-, m- oder p-Fluorphenyl, o-, m- oder p-Chlorphenyl, o-, m- oder p-Bromphenyl, o-, m- oder p-(1H-5-Tetrazolyl)-phenyl, ferner bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl.

$Het^1$ ist vorzugsweise Tetranydro-2- oder -3-furyl, Tetrahydro-2- oder -3-thienyl, 1-, 2- oder 3-Pyrrolidinyl, 2-, 3-, 4- oder 5-Oxazolidinyl, 2-, 3-, 4- oder 5-Thiazolidinyl, 1-, 2-, 3-, 4- oder 5-Imdazolidinyl, 2-, 3- oder 4-Tetrahydropyranyl, 2-, 3- oder 4-Tetrahydrothiopyranyl, 1-, 2-, 3- oder 4-Piperidinyl, 2-, 3- oder 4-Morpholinyl, 1,- 2- oder 3-Piperazinyl, 1-Methyl-2- oder -3-pyrrolidinyl, 1-Methyl-2-, -3- oder -4-piperidinyl, 4-Methyl-2- oder -3-morpholinyl, 1-Methyl-2-, -3- oder -4-piperazinyl, 1-Phenyl-2- oder -3-pyrrolidinyl, 1-Phenyl-2-, -3- oder -4-piperidinyl, -4-Phenyl-2- oder -3-morpholinyl, 1-Phenyl-2-, -3- oder 4-piperazinyl, 2-Oxo-3-, -4- oder -5-oxazolidinyl, 2-Oxo-3-, -4- oder -5-thiazolidinyl, 2-Oxo-1-, -3-, -4- oder -5-imidazolidinyl, 2,4-Dioxo-1-, -3- oder -5-imidazolidinyl, 2-Oxo-3-phenyl-4- oder -5-oxazolidinyl, 2-Oxo-3-o-, -m- oder -p-tolyl-4- oder -5-oxazolidinyl, 2-Hydroxyimino-3-, -4- oder -5-oxazolidinyl, 2-Methoxyimino-3-, -4- oder -5-oxazolidinyl, 2-Hydroxyimino-4-oxo-3- oder -5-oxazolidinyl, 2-Methoxyimino-4-oxo-3- oder -5-oxazolidinyl.

$Het^2$ ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder -5-yl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -4-yl, 2,1,5-Thiadiazol-3- oder -4-yl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2-1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolyl, 2-, 4-, 5-, 6-, 7- oder 8-Chinazolyl, 1H-1-, -2-, -5-, -6- oder -7-Imidazo[4,5-b]pyridyl, 3H-2-, -3-, -5-, -6- oder -7-Imidazo[4,5-b]pyridyl, 1H-1-, -2-, -4-, -6- oder -7-Imidazo[4,5-c]pyridyl, 3H-2-, -3-, -4-, -6- oder -7-Imidazo-[4,5-c]pyridyl.

In den Begriff "$Het^2$" eingeschlossen sind auch die homologen Reste, in denen der heteroaromatische Ring durch eine oder mehrere, vorzugsweise 1 oder 2, A-Gruppen, vorzugsweise Methyl- und/oder Ethylgruppen substituiert ist, z.B. 3-, 4- oder 5-Methyl-2-furyl, 2-, 4- oder 5-Methyl-3-furyl, 2,4-Dimethyl-3-furyl, 3-, 4- oder 5-Methyl-2-thienyl, 3-Methyl-5-tert.-butyl-2-thienyl,2-, 4- oder 5-Methyl-3-thienyl, 2- oder 3-Methyl-1-pyrrolyl, 1-, 3-, 4- oder 5-Methyl-2-pyrrolyl, 3,5-Dimethyl-4-ethyl-2-pyrrolyl, 2-, 4- oder 5-Methyl-1-imidazolyl,4-Methyl-5-pyrazolyl, 4- oder 5-Methyl-3-isooxazolyl, 3- oder 5-Methyl-4-isoxazolyl, 3- oder 4-Methyl-5-isoxazolyl, 3,4-Dimethyl-5-isoxazolyl, 4- oder 5-Methyl-2-thiazolyl, 4- oder 5-Ethyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 4-Methyl-2-pyrimidinyl, 4,5-Dimethyl-2-pyrimidinyl, 2-, 5- oder 6-Methyl-4-pyrimidinyl, 2,6-Dimethyl-4-pyrimidinyl, 3-, 4-, 5-, 6- oder 7-Methyl-2-benzofuryl, 2-Ethyl-3-benzofuryl, 3-, 4-, 5-, 6- oder 7-Methyl-2-benzothienyl, 3-Ethyl-2-benzothienyl, 1-, 2-, 4-, 5-, 6- oder 7-Methyl-3-indolyl, 1-Methyl-5- oder 6-benzimidazolyl, 1-Ethyl-5- oder 6-benzimidazolyl.

Die Gruppen $-C_kH_{2k}-$ und $-C_nH_{2n}-$ sind vorzugsweise geradkettig und stehen somit bevorzugt für $-(CH_2)_n-$ und $-(CH_2)_k-$, insbesondere für $-CH_2-$, ferner für $-CH_2CH_2-$, $-(CH_2)_3-$, $-(CH_2)_4-$, $-(CH_2)_5-$ oder $-(CH_2)_6-$, aber auch z.B. für $-CH(CH_3)-$, $-CH_2-CH(CH_3)-$ oder $-C(CH_3)_2-$. Der Parameter kann bevorzugt auch O sein, so daß die Gruppe $C_kH_{2k}-$ fehlt.

Der Rest $R^1$ ist vorzugsweise geradkettig und steht bevorzugt für A, insbesondere Ethyl, Propyl oder Butyl, ferner Methyl, Pentyl oder Hexyl, sowie für Cycloalkyl min 3-7 C-Atomen, insbesondere Cyclopropyl, ferner Cyclobutyl, Cyclopentyl, Cyclohexyl, Cycloheptyl, weiterhin insbesondere für Alkenyl mit vorzugsweise 3-6 C-Atomen, vor allem Allyl oder 1-Propenyl, ferner 1-Butenyl, 1-Pentenyl oder 1-Hexenyl; für Alkinyl mit vorzugsweise 3-6 C-Atomen, vor allem Propargyl oder 1-Propinyl, ferner 1-Butinyl, 1-Pentinyl oder 1-Hexinyl; für Cycloalkylalkyl mit vorzugsweise 4-8 C-Atomen, vor allem Cyclopropylmethyl, 1- oder 2-Cyclopropylethyl, ferner Cyclobutylmethyl, Cyclopentylmethyl, Cyclohexylmethyl; für Alkoxy mit vorzugsweise 1-4 C-Atomen wie Methoxy, Ethoxy, Propoxy, Butoxy, Isobutoxy; für Alkoxyalkyl mit vorzugsweise 2-5 C-Atomen wie Methoxymethyl, Ethoxymethyl, Propoxymethyl, 2-Methoxyethyl, 3-Methoxypropyl, 2-Ethoxyethyl; für Alkylthio mit vorzugsweise 1-4 C-Atomen wie Methylthio, Ethylthio, Propylthio, Butylthio, Isobutylthio; für Alkylthioalkyl mit vorzugsweise 2-5 C-Atomen wie Methylthiomethyl, Ethylthiomethyl, Propylthiomethyl, 2-Methylthio-ethyl, 3-Methylthiopropyl, 2-Ethylthio-ethyl.

Der Rest $R^2$ ist vorzugsweise 1H-5-Tetrazolyl, ferner bevorzugt COOH, $COOCH_3$, $COOC_2H_5$, CN oder $NHSO_2CF_3$.

Der Rest $R^3$ steht bevorzugt für $-C_nH_{2n}R^9$ (im einzelnen bevorzugt für $-CH_2R^9$), insbesondere für $-C_nH_{2n}-CO-NR^6R^7$ (im einzelnen für $-CH_2-CO-NR^6R^7$). Weitere bevorzugte Bedeutungen des Restes $R^3$ sind Ar-alkenyl (mit 2-6 C-Atomen im "alkenyl"-Teil, z.B. Cinnamyl; $-C_nH_{2n}-C(=NR^{12})-A$ [im einzelnen $-CH_2-C(=NR^{12})-A$], insbesondere $-C_nH_{2n}-C(=NOA)-A$ [im einzelnen $-CH_2-C(=NOA)-A$], z.B. 2-Methoxyimino-3,3-dimethylbutyl; im "alkenyl"-Teil durch COOA substituiertes Ar-alkenyl (mit 2-6 C-Atomen im "alkenyl"-Teil),

z.B. 3-Ethoxycarbonyl-2-phenyl-2-propen-1-yl; -CH(COOA)-Ar, z.B. $\alpha$-Methoxycarbonyl-benzyl, $\alpha$-Ethoxycarbonyl-benzyl; $-C_nH_{2n}-Het^1$ (im einzelnen $-CH_2-Het^1$), insbesondere $-C_nH_{2n}$-(2-oxo-3-Ar-5-oxazolidinyl) [im einzelnen $-CH_2$-(2-oxo-3-Ar-5-oxazolidinyl)], z.B. 2-Oxo-3-m-tolyl-5-oxazolidinyl-methyl. Einige speziellere bevorzugte Reste $R^3$ sind $-(CH_2)_t$-CO-NR'R'', worin t 1 oder 2 und R' und R'' H oder A bedeuten, z.B. Carbamoylmethyl, 2-Carbamoylethyl, N-Methylcarbamoylmethyl, 2-N-Methylcarbamoylethyl, N-Ethylcarbamoylmethyl, N-Propyl-carbamoylmethyl, N-Isopropyl-carbamoylmethyl, N-Butyl-carbamoylmethyl, N-Isobutyl-carbamoylmethyl, N-sek.-Butyl-carbamoylmethyl, N-tert.-Butyl-carbamoylmethyl, N,N-Dimethyl-carbamoylmethyl, 2-N,N-Dimethyl-carbamoylethyl, N-Methyl-N-ethyl-carbamoylmethyl, N,N-Diethyl-carbamoylmethyl, N,N-Dipropyl-carbamoylmethyl, N,N-Diisopropyl-carbamoylmethyl, N,N-Dibutyl-carbamoylmethyl; $-(CH_2)_t$-CO-NHAr, z.B. N-Phenyl-carbamoylmethyl, 2-N-Phenyl-carbamoylethyl, N-o-, -m- oder -p-Tolylcarbamoylmethyl, N-o-, m- oder -p-Trifluormethylphenyl-carbamoylmethyl, N-o-, -m- oder -p-Carboxyphenyl-carbamoylmethyl, N-o-, -m- oder -p-Ethoxycarbonylphenyl-carbamoylmethyl, N-o-, -m- oder p-Fluorphenyl-carbamoylmethyl, N-o-, -m- oder-p-Chlorphenyl-carbamoylmethyl, N-(2,3-, N-(2,4-, N-(2,5-, N-(2,6-, N-(3,4- oder N-(3,5-Dimethylphenyl)-carbamoylmethyl, 2-N-(2,3-,2-N-(2,4-, 2-N-(2,5-, 2-N-(2,6-, -2-N-(3,4- oder 2-N-(3,5-Dimethylphenyl)-carbamoylethyl;$-(CH_2)_t$-CO-NH-$Het^2$, z.B. N-(2-, N-(3- oder N-(4-Pyridyl)-carbamoylmethyl, 2-N-(2-Pyridyl)-carbamoylethyl, N-(2- oder N-(3-Thienyl)-carbamoylmethyl; $-(CH_2)_t$-CO-NAAr, z.B. N-Methyl-N-phenyl-carbamoylmethyl, 2-N-Methyl-N-phenyl-carbamoylethyl, N-Ethyl-N-phenyl-carbamoylmethyl; $-(CH_2)_t$-CO-NA($C_nH_{2n}$-Ar), z.B. N-Methyl-N-benzyl-carbamoylmethyl, N-Methyl-N-(2-phenylethyl)-carbamoylmethyl, N-Methyl-N-(1,1-dimethyl-2-phenylethyl)-carbamoylmethyl, 2-N-Methyl-N-(1,1-dimethyl-2-phenylethyl)-carbamoylethyl; $-(CH_2)_t-CO-CH_2-NO_2$, z.B. 3-Nitro-2-oxopropyl, 4-Nitro-3-oxopropyl; $-(CH_2)_t-CO-C_nH_{2n}$-NH-COOA, z.B. 4-BOC-amino-2-oxobutyl, 5-BOC-amino-2-oxopentyl, 6-BOC-amino-2-oxohexyl; $-(CH_2)_t-CO-C_nH_{2n}-NH_2$, z.B. 3-Amino-2-oxopropyl,4-Amino-2-oxobutyl, 5-Amino-2-oxopentyl, 6-Amino-2-oxohexyl, 4-Amino-3-oxobutyl; $-(CH_2)_t-CO-NH-SO_2Ar$, z.B. N-Phenylsulfonylcarbamoylmethyl; $-(CH_2)_t$-S-A, z.B. Methylthiomethyl; $-(CH_2)_t$-SO-A, z.B. Methylsulfinylmethyl; $-(CH_2)_t-SO_2$-A, z.B. Methylsulfonylmethyl; $-(CH_2)_t$-S-Ar, z.B. Phenylthio-methyl; $-(CH_2)_t$-SO-Ar, z.B. Phenylsulfinyl-methyl; $-(CH_2)_t-SO_2$-Ar, z.B. Phenylsulfonyl-methyl; $-(CH_2)_t$-S-$Het^2$, z.B. (2-Thienyl)-thiomethyl; $-(CH_2)_t$-SO-$Het^2$, z.B. (2-Pyridyl)-sulfinylmethyl; $-(CH_2)_t-SO_2-Het^2$, z.B. (2-, (3- oder (4-Pyridyl)-sulfonylmethyl.

Der Rest $R^4$ ist bevorzugt H, aber auch F, Cl, Br oder I.

Die Reste $R^5$ und $R^8$ enthalten bevorzugt 1, 2 oder 3-C-Atome und bedeuten vorzugsweise Methyl, Ethyl, Trifluormethyl, Pentafluorethyl, 2,2,2-Trifluorethyl oder 3,3,3-Trifluorpropyl. Falls eine Verbindung der Formel I zwei Reste $R^5$ enthält, so können diese gleich oder voneinander verschieden sein.

Die Reste $R^6$ und $R^7$ stehen vorzugsweise für H oder A, $R^6$ steht zusätzlich bevorzugt für Ar, Ar-$C_nH_{2n}$ oder $Het^2$.

Weitere bevorzugte Gruppen $-NR^6R^7$ sind diejenigen, in denen $R^6$ und $R^7$ zusammen eine Alkylenkette mit 2-5 C-Atomen bedeuten, die wie angegeben substituiert und/oder durch O oder durch $-NR^{16}$- unterbrochen sein kann. Besonders bevorzugte Gruppen $-NR^6R^7$ dieser Art sind z.B. Aziridino, Pyrrolidino, Piperidino, Morpholino, Piperazino, 2-Oxo-pyrrolidino, 2-Alkoxycarbonyl-pyrrolidino (worin die Alkoxygruppe 1-4 C-Atome enthält) wie 2-Methoxycarbonyl-pyrrolidino oder 2-Ethoxycarbonyl-pyrrolidino, 2- oder 3-Alkanoylamino-pyrrolidino wie 2- oder 3-Acetamido-pyrrolidino, 2-, 3- oder insbesondere 4-Oxopiperidino, 2-, 3- oder insbesondere 4-Ar-piperidino wie 2-, 3- oder 4-Phenyl-piperidino, 4-o-, 4-m- oder 4-p-Methoxyphenyl-piperidino, 4-o-, 4-m- oder 4-p-Nitrophenyl-piperidino, 4-o-, 4-m- oder 4-p-Chlorphenylpiperidino, 3-Hydroxymethyl-4-p-chlorphenyl-piperidino, 2-, 3- oder 4-(2-Thienyl)-piperidino, 2-, 3- oder 4-N,N-Dimethylcarbamoyl-piperidino, 2-, 3- oder 4-N,N-Diethylcarbamoyl-piperidino, 2-, 3- oder 4-Benzoylpiperidino, 2-, 3- oder 4-p-Methoxybenzoyl-piperidino, 4-Methylpiperazino, 4-Phenylpiperazino, 4-o-, 4-m-oder 4-p-Methoxyphenyl-piperazino, 4-o-, 4-m- oder 4-p-Nitrophenyl-piperazino, 4-o-, 4-m- oder 4-p-Chlorphenyl-piperazino, 4-(2-Pyrimidinyl)-piperazino, 4-Methoxycarbonyl-piperazino, 4-Ethoxycarbonyl-piperazino, 4-BOC-piperazino, 4-Phenylsulfonyl-piperazino, 4-p-Tolylsulfonyl-piperazino, 4-o-, 4-m- oder 4-p-Fluorphenylsulfonyl-piperazino.

$R^9$ ist vorzugsweise $-CO-NR^6R^7$, ferner bevorzugt $-CO-R^{14}$ oder $-C(=NR^{12})$-A.

$R^{10}$ ist vorzugsweise COOH oder COOA.

$R^{11}$ ist vorzugsweise Ar, inbesondere Phenyl,

$R^{12}$ ist vorzugsweise OH oder $OR^{13}$, insbesondere OA:

$R^{13}$ ist vorzugsweise A.

$R^{14}$ ist vorzugsweise $-C_nH_{2n}-NO_2$ oder $-C_nH_{2n}-NR^6R^7$ insbesondere $-C_nH_{2n}NH_2$.

$R^{15}$ ist vorzugsweise H, ferner A mit 1-4 C-Atomen.

m ist vorzugsweise O oder 2.

n ist vorzugsweise 1, ferner bevorzugt 2, 3 oder 4.

Der Rest X fehlt vorzugsweise oder bedeutet vorzugsweise -NH-CO- oder -CO-NH-.

Der Rest Y ist vorzugsweise O, aber auch S.

Die Verbindungen der Formel I können ein odere mehrere chirale Zentren besitzen und daher in verschiedenen - optisch-aktiven oder optisch-inaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ii ausgedrückt werden, die der Formel I entsprechen und worin die nicht näher bezeichneten Reste die bei Formel I angegebenen Bedeutungen haben, worin jedoch:

in Ia X fehlt;
in Ib X -NH-CO- bedeutet;
in Ic X -CO-NH- bedeutet;
in Id X -O-CH(COOH)- bedeutet;
in Ie X -NH-CH(COOH)- bedeutet;
in If X -CH = C(COOH)- bedeutet;
in Ih X -CH = C(CN)- bedeutet;
in Ii X -CH = C(1H-5-tetrazolyl)- bedeutet.

Verbindungen der Formel Ia sind besonders bevorzugt.

Weiterhin sind bevorzugt:

Verbindungen der Formeln Ik sowie Iak bis Iik, die den Verbindungen der Formeln I sowie Ia bis Ii entsprechen, worin jedoch zusätzlich Y ein O-Atom bedeutet;

Verbindungen der Formeln Il, Ial bis Ikl, sowie Iakl bis Iikl, die den Formeln I, Ia bis Ik sowie Iak bis Iik entsprechen, worin jedoch zusätzlich $R^4$ H bedeutet;

Verbindungen der Formeln Im, Iam bis Ilm, Ialm bis Iklm sowie Iaklm bis Iiklm, die den Formeln I, Ia bis Il, Ial bis Ikl sowie Iakl bis Iikl entspechen, worin jedoch zusätzlich $R^2$ CN oder 1H-5-Tetrazolyl bedeutet.

Unter diesen sind diejenigen Verbindungen bevorzugt, worin $R^1$ A oder Alkenyl mit jeweils 3-6 C-Atomen bedeutet.

Weitere bevorzugte Gruppen von Verbindungen entsprechen der Formel I sowie den anderen vorstehend genannten Formeln, worin jedoch der Rest $R^3$ folgende Bedeutungen hat:

(a) alkenyl-Ar mit 2-6 C-Atomen im "alkenyl"-Teil,
(b) $-C_nH_{2n}-R^9$
(c) $-CHR^{10}$-Ar,
(d) $-C_nH_{2n}-CO-NR^6R^7$,
(e) $-(CH_2)_t$-CO-NR'R'' (worin t 1 oder 2, R' und R'' jeweils H oder A bedeuten),
(f) $-CH_2-CO-NR^6R^7$, worin $R^6$ und $R^7$ zusammen eine Alkylenkette mit 2-5 C-Atomen bedeuten, die ein- oder mehrfach durch Carbonylsauerstoff, Ar, $Het^2$, -CO-Ar, -COOA, $-CO-N(A)_2$, $-CH_2OH$, $-SO_2$-Ar und/oder -NH-CO-A substituiert und/oder durch O oder durch $-NR^{16}$- unterbrochen sein kann,
(g) $-CH_2-CO-NR^6R^7$, worin $-NR^6R^7$ Pyrrolidino, Piperidino, Morpholino, 3-Acetamido-pyrrolidino, 4-Oxopiperidino, 3-Diethylaminocarbonyl-piperidino, 4-o-Methoxyphenylpiperidino, 4-o-Methoxyphenyl-piperazino, 4-o-Nitrophenyl-piperazino oder 4-p-Fluorphenylsulfonyl-piperazino bedeutet.

Eine kleine ausgewählte Gruppe bevorzugter Verbindungen entspricht der Formel I, worin

R einen 2-Butyl-4,5-dihydro-4-oxo-5-$R^3$-3H-imidazo[4,5-c]pyridin-3-yl-rest,
$R^2$ 1H-5-Tetrazolyl und
$R^3$ $-C_nH_{2n}-CONR^6R^7$ bedeuten und
X fehlt.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der EP-A2-O 430 709 und in der US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt. Besonders die Biphenylderivate der Formel I (worin X fehlt) sind auf diesem Wege gut erhältlich.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder

Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie $CH_3ONa$ oder K-tert.-Butylat in einem Alkohol wie Methanol oder tert.-Butanol oder mit einem Alkalimetallhydrid wie NaH oder einem Alkalimetallalkoholat in Dimethylformamid (DMF), und dieses dann in einem inerten Lösungsmittel, z.B. einem Amid wie DMF, N-Methylpyrrolidon oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°. Als Basen eignen sich auch Alkalimetall-hydrogencarbonate wie $NaHCO_3$ oder $KHCO_3$.

Die Verbindungen der Formel I sind weiterhin durch Cyclisierung von Verbindungen der Formel IV erhältlich. Diese Cyclisierung gelingt zweckmäßig durch Erhitzen mit Polyphosphorsäure, Essigsäure oder Diglyme auf Temperaturen zwischen etwa 80 und 180°, vorzugsweise zwischen 120 und 160°.

Säureamide der Formel I (X = -NH-CO- oder -CO-NH) sind ferner erhältlich durch Umsetzung von Verbindungen der Formel V (oder ihrer reaktionsfähigen Derivate) mit Verbindungen der Formel VI (oder ihrer reaktionsfähigen Derivate).

Als reaktionsfähige Derivate der Carbonsäuren der Formeln V und VI ($X^1$ bzw. $X^2$ = COOH) eignen sich vorteilhaft die entsprechenden Chloride, Bromide oder Anhydride. Die Umsetzung erfolgt zweckmäßig in Gegenwart eines inerten Lösungsmittel, z.B. eines halogenierten Kohlenwasserstoffs wie Dichlormethan, Chloroform, Trichlorethen oder 1,2-Dichlorethan oder eines Ethers wie Tetrahydrofuran (THF) oder Dioxan, bei Temperaturen zwischen 0 und 150°, vorzugsweise zwischen 20 und 80°. Setzt man Säurehalogenide um, so empfiehlt sich der Zusatz einer Base, z.B. eines tertiären Amins wie Triethylamin, Pyridin oder 4-Dimethylaminopyridin.

Die Verbindungen der Formel I können auch durch Reaktion einer Verbindung der Formel VII (entsprechend der Formel I, aber H an Stelle von $R^3$) mit einer Verbindung der Formel VIII erhalten werden. Man arbeitet dabei bevorzugt in einem inerten Lösungsmittel, z.B. einem Säureamid wie DMF, N-Methylpyrrolidon, 1,3-Dimethyl-2-oxo-hexahydropyrimidin oder Phosphorsäure-hexamethyl-triamid, einem Alkohol wie Methanol oder tert.-Butanol, einem Ether wie THF oder einem halogenierten Kohlenwasserstoff wie Dichlormethan oder Gemischen davon als Lösungsmittel und/oder in Gegenwart eines Alkalimetallalkoholats wie Natriummethylat oder Kalium-tert.-butylat, eines Alkalimetallhydrids wie Natrium- oder Kaliumhydrid, eines Alkalimetallcarbonats wie Natrium- oder Kaliumcarbonat, eines Alkalimetallbicarbonats wie Natrium- oder Kaliumbicarbonat oder eines tertiären Amins wie Triethylamin oder Ethyldiisopropylamin bei Temperaturen zwischen etwa -30 und 200, vorzugsweise zwischen 20 und 60°.

Verbindungen der Formel I, welche die Gruppe $-C(=NR^{12})$-enthalten, können aus Carbonylverbindungen, die stattdessen die Gruppe -CO- enthalten, sonst aber der Formel I entsprechen, durch Umsetzung mit einer Verbindung der Formel $H_2N-R^{12}$ hergestellt werden. Diese letztgenannten umfassen Ammoniak, Hydroxylamin, O-Alkyl-, O-Alkenyl-, O-Alkinyl- und O-Arylhydroxylamine, Cyanamid und primäre Amine der Formel $R^{13}-NH_2$.

Verbindungen der Formel I, in denen $R^3$ $-C_nH_{2n}-CO-R^{19}$ bedeutet, können auch erhalten werden durch Umsetzung von Carbonsäuren, die der Formel I entsprechen, aber an Stelle des Restes $R^3$ eine $-C_nH_{2n}-COOH$-Gruppe enthalten, mit Aminen der Formel $H-R^{19}$. Dabei arbeitet man zweckmäßig nach üblichen Methoden der Peptid-Synthese, wie sie z.B. in Houben-Weyl, 1.c., Band 15/II, Seiten 1-806 (1974) beschrieben sind.

Die Reaktion gelingt vorzugsweise in Gegenwart eines Dehydratisierungsmittels, z.B. eines Carbodiimids wie N,N'-Dicyclohexylcarbodiimid ("DCCI"), 1,1'-Carbonyl-diimidazol oder N-3-Dimethylaminopropyl-N'-ethyl-carbodiimid ("DAPECI"), ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle der Carbonsäuren können auch geeignete reaktionsfähige Derivate dieser Stoffe in die Reaktion eingesetzt werden, z.B. solche, in denen reaktive Gruppen intermediär durch Schutzgruppen blockiert sind. Die Säuren können z.B. in Form ihrer aktivierten Ester verwendet werden, die zweckmäßig in situ gebildet werden, z.B. durch Zusatz von 1-Hydroxy-benzotriazol oder N-Hydroxysuccinimid.

Weiterhin kann man eine Verbindung der Formel I durch Solvolyse (z.B. Hydrolyse) oder Hydrogenolyse aus einem ihrer funktionellen Derivate in Freiheit setzen.

So kann man Carbonsäuren der Formel I, worin X -O-CH(COOH)-, -NH-CH(COOH)-, -NA-CH(COOH)- oder -CH=C(COOH)- bedeutet, erhalten durch Verseifung entsprechender Alkylester, z.B. mit NaOH oder KOH in wässeriger Lösung mit oder ohne Zusatz eines inerten organischen Lösungsmittels wie Methanol, Ethanol, THF oder Dioxan bei Temperaturen zwischen 0 und 100°, oder durch Hydrogenolyse entsprechender Benzylester, z.B. an Pd-Kohle bei Drucken zwischen 1 und 200 bar und bei Temperaturen zwischen 0 und 100° in einem der angegebenen inerten Lösungsmittel.

Ferner ist es möglich, nach einer der angegebenen Methoden eine Verbindung herzustellen, die der Formel I entspricht, aber an Stelle einer 5-Tetrazolylgruppe eine in 1-Stellung (oder 2-Stellung) funktionell abewandelte (durch eine Schutzgruppe geschützte) 1H-(oder 2H)-5-Tetrazolylgruppe enthält. Als Schutzgruppe eignen sich beispielsweise: Triphenylmethyl, abspaltbar mit HCl oder Ameisensäure in einem inerten Lösungsmittel oder Lösungsmittelgemisch, z.B. Ether/Dichlormethan/Methanol; 2-Cyanethyl, abspaltbar mit NaOH in Wasser/THF; p-Nitrobenzyl, abspaltbar mit $H_2$/Raney-Nickel in Ethanol (vgl.EP-A2-O 291 969).

Die Ausgangsstoffe, insbesondere diejenigen der Formeln II, VI und VIII, sind teilweise bekannt. Falls sie nicht bekannt sind, können sie nach bekannten Methoden in Analogie zu bekannten Stoffen hergestellt werden. Verbindungen der Formel III (Y = O) sind z.B. erhältlich durch Reaktion von Carbonsäuren der Formel $R^1$-COOH mit Verbindungen der Formel IX

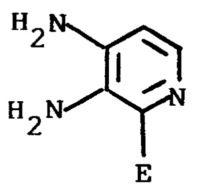

IX

in Gegenwart von Polyphosphorsäure; dabei wird die Gruppe E (vorzugsweise Cl) hydrolysiert, und es entstehen zunächst Verbindungen entsprechend Formel III, aber $R^3$ = H, die anschließend mit Verbindungen der Formel VIII umgesetzt werden.

Verbindungen der Formel IV sind z.B. erhältlich durch Umsetzung von Verbindungen der Formel X

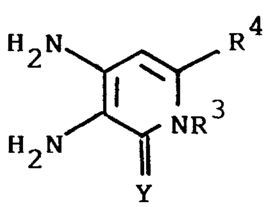

X

worin jedoch die eine Aminogruppe durch eine Aminoschutzgruppe (z.B. Benzyl, A-O-CO- oder Benzyloxycarbonyl) geschützt ist, mit Verbindungen der Formel II sowie nachfolgende Abspaltung der Schutzgruppe und Reaktion mit Säuren der Formel $R^1$-COOH oder deren funktionellen Derivaten; sie werden in der Regel nicht isoliert, sondern entstehen in situ bei der letztgenannten Umsetzung.

Verbindungen der Formel V können durch Reaktion von III mit Benzylchloriden der Formel Cl-$CH_2$-p-$C_6H_4$-$X^3$ (worin $X^3$ eine geschützte $NH_2$- oder COOH-Gruppe bedeutet) und nachfolgende Abspaltung der Schutzgruppe hergestellt werden.

Verbindungen der Formel VII sind z.B. erhältlich durch Reaktion von Verbindungen entsprechend Formel III, die jedoch an Stelle von $R^3$ ein H-Atom tragen, mit Verbindungen der Formel II.

Es ist ferner möglich, eine Verbindung der Formel I in eine andere Verbindung der Formel I umzuwandeln, indem man einen oder mehrere der Reste R und/oder $R^2$ in andere Reste R und/oder $R^2$ umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Nitrilgruppen zu COOH-Gruppen hydrolysiert oder mit Derivaten der Stickstoffwasserstoffsäure, z.B. Natriumazid in N-Methylpyrrolidon oder Trimethylzinnazid in Toluol, zu Tetrazolylgruppen umsetzt und/oder Thioethergruppen zu SO- oder $SO_2$-Gruppen oxydiert, z.B. mit $H_2O_2$ oder einer Persäure wie 3-Chlorperbenzoesäure.

So kann man beispielsweise freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z.B. eine Verbindung der Formel I, die eine $NHCOR^5$- oder eine COOA-Gruppe enthält, in die entsprechende Verbindung der Formel I umgewandelt werden, die stattdessen eine $NH_2$- oder eine HOOC-Gruppe enthält. COOA-Gruppen können z.B. mit NaOH oder KOH in Wasser, Wasser-THF oder Wasser-Dioxan bei Temperaturen zwischen 0 und 100° verseift werden.

Umsetzung von Nitrilen der Formel I (z.B. solche mit $R^2$ = CN) mit Derivaten der Stickstoffwasserstoffsäure führt zu Tetrazolen der Formel I (z.B. mit $R^2$ = 1H-5-Tetrazolyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°. Anschließend wird die Trialkylzinn-Gruppe abgespalten, entweder durch Behandeln mit Salzsäure, z.B. in Dioxan, oder mit Alkali, z.B. in Ethanol/Wasser, oder mit Ameisensäure z.B. in Methanol, oder durch Chromatographie an einer Kieselgel-Säule, z.B. mit Ethylacetat/Methanol.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure; 2-Hydroxyethansulfonsäure, Benzolsulfonsäure, p-Toluolsulfonsäure, Naphthalinmono- und disulfonsäuren, Laurylschwefelsäure. Salze mit physiologisch nicht unbedenklichen Säuren, z.B. Pikrate, können zur Isolierung und/oder Aufreinigung der Verbindung der Formel I verwendet werden.

Andererseits können Verbindungen der Formel I, die COOH- oder Tetrazolylgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze der Tetrazolylderivate sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind spezielle Lacktabletten und Kapseln mit magensaftresistenten Überzügen bzw. Kapselhüllen. Zur rektalen Anwendung dienen-Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der US-PS 4 880 804 beschriebenen Verbindungen verabreicht, vorzugsweise in Dosierungen zwischen etwa 1 mg und 1 g, insbesondere zwichen 50 und 500 mg pro Dosierungseinheit. Die tägliche Dosierung liegt vorzugsweise zwischen etwa 0,1 und 50 mg/kg, insbesondere 1 und 10 mg/kg Körpergewicht. Die spezielle Dosis für jeden bestimmten

Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation. IP = Imidazo[4,5-c]pyridin.

Beispiel 1

(a) Eine Lösung von 0,23 g Na in 20 ml Methanol wird innerhalb 15 Minuten zugetropft zu einer Lösung von 2,76 g 2-Butyl-5-(N,N-dimethylcarbamoylmethyl)-4,5-dihydro-4-oxo-3H-IP [erhältlich durch Kondensation von Valeriansäure mit 3,4-Diamino-2-chlorpyridin in Gegenwart von Polyphosphorsäure zu 2-Butyl-4,5-dihydro-4-oxo-1(oder 3)H-IP, Reaktion mit Benzylbromid in Methanol in Gegenwart von $CH_3ONa$ zu 3-Benzyl-2-butyl-4,5-dihydro-4-oxo-3H-IP, Reaktion mit N,N-Dimethyl-chloracetamid in DMF in Gegenwart von K-tert.-Butylat zu 3-Benzyl-2-butyl-5-(N,N-dimethylcarbamoylmethyl)-4,5-dihydro-4-oxo-3H-IP und hydrogenolytische Abspaltung der Benzylgruppe] in 75 ml Methanol. Man rührt noch 30 Minuten bei 20°, dampft ein, löst den Rückstand in 20 ml DMF und tropft bei 0° unter Rühren eine Lösung von 3,05 g 4'-Brommethyl-biphenyl-2-carbonsäure-methylester (IIa) in 10 ml DMF hinzu. Man rührt 16 Stunden bei 20°, dampft ein, arbeitet wie üblich auf, chromatographiert an Kieselgel und erhält 2-Butyl-5-(N,N-dimethylcarbamoylmethyl)-4,5-dihydro-3-(2'-methoxycarbonyl-biphenylyl-4-methyl)-4-oxo-3H-IP.

(b) Ein Gemisch von 1 g des nach (a) erhaltenen Methylesters, 12 ml wässeriger 2 n NaOH-Lösung und 48 ml Methanol wird 2 Std. gekocht, dann eingedampft. Man arbeitet wie üblich auf (wässeriger Salzsäure bis pH 3/Dichlormethan) und erhält 2-Butyl-5-(N,N-dimethylcarbamoylmethyl)-4,5-dihydro-3-(2'carboxy-biphenylyl-4-methyl)-4-oxo-3H-IP.

Beispiel 2

Analog Beispiel 1 erhält man aus 2,76 g 2-Butyl-4,5-dihydro-4-oxo-5-(N,N-dimethylcarbamoylmethyl)-3H-IP und 2,98 g 3-p-Brommethyl-phenyl-2-phenyl-acrylnitril [F. 178°; erhältlich durch Kondensation von p-Tolylaldehyd mit Phenylacetonitril in Gegenwart von $C_2H_5ONa$ in Ethanol zu 2-Phenyl-3-p-tolyl-acrylnitril (F. 61°) und Bromierung mit N-Bromsuccinimid in Dichlormethan] das 2-Butyl-3-[p-(1-cyan-2-phenyl-vinyl)-benzyl]-4,5-dihydro-4-oxo-5-(N,N-dimethylcarbamoylmethyl)-3H-IP.

Beispiel 3

Ein Gemisch von 1,02 g Valeriansäure, 4,44 g 4-Amino-1,2-dihydro-2-oxo-3-[2'-(1H-5-tetrazolyl)-biphenylyl-4-methylamino]-1-(N,N-dimethylcarbamoylmethyl)-pyridin [erhältlich durch Reaktion von 3-Amino-4-benzylamino-1,2-dihydro-2-oxo-1-(N,N-dimethylcarbamoylmethyl)-pyridin mit 4-Brommethyl-2'-cyan-biphenyl zu 4-Benzylamino-3-(2'-cyan-biphenylyl-4-methyl-amino)-1,2-dihydro-2-oxo-1-(N,N-dimethylcarbamoylmethyl)-pyridin, Reaktion mit Trimethylzinnazid zu 4-Benzylamino-1,2-dihydro-2-oxo-3-[2'-(1H-5-tetrazolyl)-biphenylyl-4-methyl-amino]-1-(N,N-dimethylcarbamoylmethyl)-pyridin und hydrogenolytische Abspaltung der Benzylgruppe] und 50 g Polyphosphorsäure wird 5 Stunden auf 140° erhitzt. Als Zwischenprodukte entstehen in situ 4-Amino-1,2-dihydro-2-oxo-3-[N-(2'-(1H-5-tetrazolyl)-biphenylyl-4-methyl)-N-valerylamino]-1-(N,N-dimethylcarbamoylmethyl)-pyridin und 1,2-Dihydro-2-oxo-3-[2'-(1H-5-tetrazolyl)-biphenylyl-4-methyl-amino]-1-(N,N-dimethylcarbamoylmethyl)-4-valerylamino-pyridin. Man kühlt ab, gießt auf Eis, macht mit Natronlauge alkalisch, arbeitet wie üblich auf und erhält 2-Butyl-4, 5-dihydro-5-(N,N-dimethylcarbamoylmethyl)-4-oxo-3-[2'-(1H-5-tetrazolyl)-biphenylyl-4-methyl]-3H-IP, F. 258°.

Beispiel 4

Ein Gemisch von 1,1 g 3-p-Aminobenzyl-2-butyl-4,5-dihydro-5-(N,N-dimethylcarbamoylmethyl)-4-oxo-3H-IP [erhältlich durch Reaktion von 2-Butyl-4,5-dihydro-5-(N,N-dimethylcarbamoylmethyl)-4-oxo-3H-IP mit p-Nitrobenzylbromid zu 2-Butyl-4,5-dihydro-5-(N,N-dimethylcarbamoylmethyl)-3-p-nitrobenzyl-4-oxo-3H-IP und anschließende Hydrierung], 0,6 g Phthalsäureanhydrid und 40 ml $CHCl_3$ wird 16 Stunden bei 20°

gerührt. Das ausgefallene 2-Butyl-3-[4-(o-carboxybenzamido)-benzyl]-4,5-dihydro-5-(N,N-dimethylcarbamoylmethyl)-4-oxo-3H-IP wird abfiltriert.

Beispiel 5

Ein Gemisch von 3,81 g 3-p-Aminobenzyl-2-butyl-4,5-dihydro-5-(N,N-dimethylcarbamoylmethyl)-4-oxo-3H-IP, 3 ml Triethylamin, 0,5 g 4-Dimethylaminopyridin und 120 ml Dichlormethan wird auf 5° gekühlt und tropfenweise mit einer Lösung von 2,88 g o-Trifluormethansulfonamidobenzoylchlorid in 20 ml Dichlormethan versetzt. Man rührt noch 16 Stunden bei 20°, dampft ein, arbeitet wie üblich auf und erhält 2-Butyl-4,5-dihydro-5-(N,N-dimethylcarbamoylmethyl)-4-oxo-3-[4-(o-trifluormethansulfonamido-benzamido)-benzyl]-3H-IP.

Beispiel 6

Ein Gemisch von 4,10 g 2-Butyl-3-p-carboxybenzyl-4,5-dihydro-5-(N,N-dimethylcarbamoylmethyl)-4-oxo-3H-IP, 12 g Thionylchlorid und 35 ml $CHCl_3$ wird 6 Stunden gekocht und eingedampft. Das erhaltene rohe Säurechlorid wird durch mehrfaches Lösen in Toluol und Eindampfen von Thionylchlorid-Resten befreit und in 80 ml THF gelöst. Man tropft diese Lösung zu einer Lösung von 1,7 g Anthranilsäure und 0,8 g NaOH in 100 ml Wasser, rührt 24 Stunden und säuert mit Salzsäure bis pH 5 an. Nach üblicher Aufarbeitung erhält man 2-Butyl-3-[p-(2-carboxyanilino-carbonyl)-benzyl]-4,5-dihydro-5-(N,N-dimethylcarbamoylmethyl)-4-oxo-3H-IP.

Beispiel 7

(a) Eine Lösung von 3,1 g 2-Butyl-3-(2'-cyan-biphenylyl-4-methyl)-4,5-dihydro-4-oxo-3H-IP (F. 179-180°; erhältlich aus 2-Butyl-4,5-dihydro-4-oxo-1(oder 3)H-IP mit 4'-Brommethyl-2-cyan-biphenyl in DMF in Gegenwart von $K_2CO_3$) in 35 ml DMF wird unter Rühren bei 20° mit 1,25 g K-tert.-Butylat versetzt. Nach 45 Minuten Rühren wird eine Lösung von 2,54 g N,N-Dimethyl-chloracetamid in 25 ml DMF zugetropft. Man rührt noch 16 Stunden bei 20°, arbeitet wie üblich auf und erhält 2-Butyl-3-(2'-cyan-biphenylyl-4-methyl)-4,5-dihydro-5-(N,N-dimethylcarbamoylmethyl)-4-oxo-3H-IP.

Analog erhält man die nachstehenden 2-Butyl-3-(2'-cyanbiphenylyl-4-methyl)-4,5-dihydro-4-oxo-5-$R^3$-3H-IP:

mit Cinnamylbromid:
-5-cinnamyl- [ = 5-(3-phenyl-2-propen-1-yl)-]
mit 3-Ethoxycarbonyl-2-phenyl-2-propen-1-ylbromid ( = $\beta$ = Brommethylzimtsäureethylester):
-5-(3-ethoxycarbonyl-2-phenyl-2-propen-1-yl)-
mit $\alpha$-Brom-phenylessigsäure-methylester:
-5-($\alpha$-methoxycarbonyl-benzyl)-
mit 2-Methoxyimino-3,3-dimethylbutylbromid:
-5-(2-methoxyimino-3,3-dimethylbutyl)-
mit 2-Oxo-3-m-tolyl-5-oxazolinyl-methylbromid:
-5-(2-oxo-3-m-tolyl-5-oxazolinyl-methyl)-
mit Bromacetamid:
-5-carbamoylmethyl-, F.219°
mit N-Methyl-chloracetamid:
-5-(N-methyl-carbamoylmethyl)-
mit N-Ethyl-chloracetamid:
-5-(N-ethyl-carbamoylmethyl)-
mit N-tert.Butyl-chloracetamid:
-5-(N-tert.-butyl-carbamoylmethyl)-
mit N,N-Diethyl-chloracetamid:
-5-(N,N-diethyl-carbamoylmethyl)-
mit N,N-Diisobutyl-chloracetamid:
-5-(N,N-diisobutyl-carbamoylmethyl)-, F. 170°
mit N-Phenyl-chloracetamid:
-5-(N-phenyl-carbamoylmethyl)-, F. 107°
mit N-o-Tolyl-chloracetamid:
-5-(N-o-tolyl-carbamoylmethyl)-, F. 175°

mit N-o-Trifluormethylphenyl-chloracetamid:
-5-(N-o-trifluomethylphenyl-carbamoylmethyl)-
mit N-o-Ethoxycarbonylphenyl-chloracetamid:
-5-(N-o-ethoxycarbonylphenyl-carbamoylmethyl)-
mit N-o-Chlorphenyl-chloracetamid:
-5-(N-o-chlorphenyl-carbamoylmethyl)-
mit N-(2,6-Dimethylphenyl)-chloracetamid:
-5-[N-(2,6-dimethylphenyl)-carbamoylmethyl]-
mit N-(2-Pyridyl)-chloracetamid:
-5-[N-(2-pyridyl)-carbamoylmethyl]-
mit N-Methyl-N-phenyl-chloracetamid:
-5-(N-methyl-N-Phenyl-carbamoylmethyl)-, F. 106°
mit N-Methyl-N-(1,1-dimethyl-2-phenylethyl)-chloracetamid:
-5-[N-methyl-N-(1,1-dimethyl-2-phenylethyl)-carbamoylmethyl]-
mit N,N-Diphenyl-chloracetamid:
-5-(N,N-diphenyl-carbamoylmethyl)-
mit 3-Chloryropionamid:
-5-(2-carbamoylethyl)-
mit 3-Chlor-N,N-dimethyl-propionamid:
-5-(2-N,N-dimethyl-carbamoylethyl)-
mit 3-Chlor-N-phenyl-propionamid:
-5-(2-N-phenyl-carbamoylethyl)-
mit 3-Chlor-N-(2,6-dimethylphenyl)-propionamid:
-5-[2-N-(2,6-dimethylphenyl)-carbamoylethyl]-
mit 1-Chlor-3-nitro-aceton:
-5-(3-nitro-2-oxo-propyl)-
mit 6-BOC-amino-1-chlor-2-hexanon:
-5-(6-BOC-amino-2-oxo-hexyl)-
mit Chloressigsäure-N-ethoxycarbonylmethyl-N-methyl-amid:
-5-(N-ethoxycarbonylmethyl-N-methyl-carbamoylmethyl)-
mit Chloressigsäure-N-(methylsulfonyl)-amid:
-5-(N-methylsulfonyl-carbamoyl-methyl)-
mit Chloressigsäure-N-(phenylsulfonyl)-amid:
-5-(N-phenylsulfonyl-carbamoyl-methyl)-, F. 193°
mit Chloressigsäure-aziridid:
-5-aziridinocarbonylmethyl-
mit Chloressigsäure-pyrrolidid:
-5-pyrrolidinocarbonylmethyl-, F. 166°
mit Chloressigsäure-piperidid:
-5-piperidinocarbonylmethyl-
mit Chloressigsäure-(2-oxo-pyrrolidid):
-5-(2-oxopyrrolidinocarbonylmethyl)-
mit Chloressigsäure-(2-oxo-piperidid):
-5-(2-oxopiperidinocarbonylmethyl)-
mit Chloressigsäure-(4-oxo-piperidid):
-5-(4-oxopiperidinocarbonylmethyl)-
mit Chloressigsäure-(4-o-methoxyphenyl-piperidid):
-5-(4-o-methoxyphenyl-piperidinocarbonylmethyl)-
mit Chloressigsäure-[4-(2-thienyl)-piperidid]:
-5-[4-(2-thienyl)-piperidinocarbonylmethyl]-
mit Chloressigsäure-(4-p-methoxybenzoyl-piperidid):
-5-(4-p-methoxybenzoyl-piperidinocarbonylmethyl)-
mit Chloressigsäure-(2-ethoxycarbonyl-pyrrolidid):
-5-(2-ethoxycarbonyl-pyrrolidinocarbonylmethyl)-
mit Chloressigsäure-(3-ethoxycarbonyl-pipridid):
-5-(3-ethoxycarbonyl-piperidinocarbonylmethyl)-
mit Chloressigsäure-(3-hydroxymethyl-4-p-chlorphenyl-piperidid):
-5-(3-hydroxymethyl-4-p-chlorphenyl-piperidinocarbonylmethyl)-

mit Chloressigsäure-(3-N,N-diethylcarbamoyl-piperidid):
-5-(3-N,N-diethylcarbamoyl-piperidinocarbonylmethyl)-, F. 97°
mit Chloressigsäure-(3-acetamido-pyrrolidid):
-5-(3-acetamido-pyrrolidinocarbonylmethyl)-
mit Chloressigsäure-morpholid:
-5-morpholinocarbonylmethyl-
mit Chloressigsäure-(3-oxo-piperazid):
-5-(3-oxo-piperazinocarbonylmethyl)-
mit Chloressigsäure-(4-methylpiperazid):
-5-(4-methylpiperazinocarbonylmethyl)-
mit Chloressigsäure-(4-o-methoxyphenyl-piperazid):
-5-(4-o-methoxyphenyl-piperazinocarbonylmethyl)-
mit Chloressigsäure-(4-o-nitrophenyl-piperazid):
-5-(4-o-nitrophenyl-piperazinocarbonylmethyl)-
mit Chloressigsäure-(3-ethoxycarabonyl-piperazid):
-5-(3-ethoxycarbonyl-piperazinocarbonylmethyl)-
mit Chloressigsäure-(4-BOC-piprazid):
-5-(4-BOC-piperazinocarbonylmethyl)-
mit Chloressigsäure-[4-(2-pyrimidinyl)-piperazid]:
-5-[4-(2-pyrimidinyl)-piperazinocarbonylmethyl]-
mit Chloressigsäure-(4-p-fluorphenylsulfonyl-piperazid):
-5-(4-p-fluorphenylsulfonyl-piperazinocarbonylmethyl)-, F. 207°
mit Methylthiomethylchlorid:
-5-methylthiomethyl-
mit Methylsulfinylmethylchlorid:
-5-methylsulfinylmethyl-
mit Methylsulfonylmethylchlorid:
-5-methylsulfonylmethyl-
mit Phenylthiomethylchlorid:
-5-phenylthiomethyl, F. 105°
mit Phenylsulfinylmethylchlorid:
-5-phenylsulfinylmethyl-, F. 115°
mit Phenylsulfonylmethylbromid:
-5-phenylsulfonylmethyl-
mit 2-Thienyl-thiomethylchlorid:
-5-(2-thienylthiomethyl)-
mit 2-Pyridylaminosulfonylmethylchlorid:
-5-(2-pyridylaminosulfonylmethyl)-
mit Methoxysulfonylmethylchlorid:
-5-methoxysulfonylmethyl-.

(b) Ein Gemisch von 3,95 g der nach (a) erhaltenen Verbindung, 20,6 g Trimethylzinnazid und 200 ml Toluol wird 24 Stunden gekocht und daan eingedampft. Man nimmt den Rückstand in 100 ml methanolischer HCl auf, rührt 2 Stunden bei 20° und arbeitet wie üblich auf (gesättigte NaCl-Lösung/Dichlormethan). Chromatographie (Ethylacetat/Hexan 80:20) liefert 2-Butyl-4,5-dihydro-5-(N,N-dimethyl-carbamoyl-methyl)-4-oxo-3-[2'-(1H-5-tetrazolyl)-biphenylyl-4-methyl]-3H-IP, F. 258°. K-Salz, Monohydrat, F. 296°.

Analog erhält man aus den unter (a) angegebenen 2'-Cyanbiphenylyl-Verbindungen die nachstehenden 2-Butyl-4,5-dihydro-4-oxo-3-[2'-(1H-5-tetrazolyl)-biphenylyl-4-methyl]-5-$R^3$-3H-IP:
-5-cinnamyl-, F. 144°
-5-(3-ethoxycarbonyl-2-phenyl-2-propen-1-yl)-, F. 163°
-5-($\alpha$-methoxycarbonyl-benzyl)-, F. 208°
-5-(2-methoxyimino-3,3-dimethyl-butyl)-, F. 83°
-5-(2-oxo-3-m-tolyl-5-oxazolidinyl-methyl)-, F. 206°
-5-carbamoylmethyl, F.115-116°
-5-(N-methyl-carbamoylmethyl)-
-5-(N-ethyl-carbamoylmethyl)-
-5-(N-tert.-butyl-carbamoylmethyl)-, Tetrahydrat, F. 212°
-5-(N,N-diethyl-carbamoylmethyl)-, Dihydrat, F. 255°

-5-(N,N-diisobutyl-carbamoylmethyl)-, Monohydrat, F. 180°
-5-(N-phenyl-carbamoylmethyl)-, Monohydrat, F. 227°
-5-(N-o-tolyl-carbamoylmethyl)-, F. 163°
-5-(N-o-trifluormethylphenyl-carbamoylmethyl)-
-5-(N-o-ethoxycarbonylphenyl-carbamoylmethyl)-, F. 190°
-5-(N-o-chlorphenyl-carbamoylmethyl)-
-5-[N-(2,6-dimethylphenyl)-carbamoylmethyl]-, Pentahydrat, F. 247°
-5-[N-(2-pyridyl)-carbamoylmethyl]-
-5-(N-methyl-N-phenyl-carbamoylmethyl)-, F. 171°
-5-[N-methyl-N-(1,1-dimethyl-2-phenylethyl)-carbamoylmethyl]-
-5-(N,N-diphenyl-carbamoylmethyl)-, Dihydrat, F. 212°
-5-(2-carbamoylethyl)-
-5-(2-N,N-dimethyl-carbamoylethyl)-
-5-(2-N-phenyl-carbamoylethyl)-
-5-[2-N-(2,6-dimethylphenyl)-carbamoylethyl]-
-5-(3-nitro-2-oxo-propyl)-
-5-(6-BOC-amino-2-oxo-hexyl)-
-5-(N-ethoxycarbonylmethyl-N-methyl-carbamoylmethyl)-
-5-(N-methylsulfonyl-carbamoylmethyl)-
-5-(N-phenylsulfonyl-carbamoylmethyl)-
-5-aziridinocarbonylmethyl-
-5-pyrrolidinocarbonylmethyl-, F. 215°
-5-piperidinocarbonylmethyl-
-5-(2-oxopyrrolidinocarbonylmethyl)-
-5-(2-oxopiperidinocarbonylmethyl)-
-5-(4-oxopiperidinocarbonylmethyl)-
-5-(4-o-methoxyphenyl-piperidinocarbonylmethyl)-
-5-[4-(2-thienyl)-piperidinocarbonylmethyl]-
-5-(4-p-methoxybenzoyl-piperidinocarbonylmethyl)-
-5-(2-ethoxycarbonyl-pyrrolidinocarbonylmethyl)-
-5-(3-ethoxycarbonyl-piperidinocarbonylmethyl)-
-5-(3-hydroxymethyl-4-p-chlorphenyl-piperidinocarbonyl-methyl)-
-5-(3-N,N-diethylcarbamoyl-piperidinocarbonylmethyl)-, F. 173°
-5-(3-acetamido-pyrrolidinocarbonylmethyl)-
-5-morpholinocarbonylmethyl-
-5-(3-oxo-piperazinocarbonylmethyl)-
-5-(4-methylpiperazinocarbonylmethyl)-
-5-(4-o-methoxyphenyl-piperazinocarbonylmethyl)-
-5-(4-o-nitrophenyl-piperazinocarbonylmethyl)-
-5-(3-ethoxycarbonyl-piperazinocarbonylmethyl)-
-5-(4-BOC-piperazinocarbonylmethyl)-
-5-[4-(2-pyrimidinyl)-piperazinocarbonylmethyl]-
-5-(4-p-fluorphenylsulfonyl-piperazinocarbonylmethyl)-, F. 248°
-5-methylthiomethyl-
-5-methylsulfinylmethyl-
-5-methylsulfonylmethyl-
-5-phenylthiomethyl-
-5-phenylsulfinylmethyl-
-5-phenylsulfonylmethyl-
-5-(2-thienyl)-thiomethyl-
-5-(2-pyridylaminosulfonylmethyl)-
-5-methoxysulfonylmethyl-.

Beispiel 8

(a) Analog Beispiel 7 (a) erhält man aus 2-Ethyl-3-(2'-cyanbiphenylyl-4-methyl)-4,5-dihydro-4-oxo-3H-IP (F. 230°; erhältlich aus 2-Ethyl-4,5-dihydro-4-oxo-1 (oder 3)H-IP mit 4'-Brommethyl-2-cyan-biphenyl) und den in Beispiel 7 (b) angegebenen Verbindungen der Formel E-$R^3$ die nach stehenden 2-Ethyl-3-(2'-cyan-biphenylyl-4-methyl)-4,5-dihydro-4-oxo-5-$R^3$-3H-IP:
-5-cinnamyl-
-5-(3-ethoxycarbonyl-2-phenyl-2-propen-1-yl)-
-5-(α-methoxycarbonyl-benzyl)-
-5-(2-methoxyimino-3,3-dimethyl-butyl)-
-5-(2-oxo-3-m-tolyl-5-oxazolidinyl-methyl)-
-5-carbamoylmethyl-
-5-(N-methyl-carbamoylmethyl)-
-5-(N-ethyl-carbamoylmethyl)-
-5-(N-tert.-butyl-carbamoylmethyl)-
-5-(N,N-dimethyl-carbamoylmethyl)-, F. 234°
-5-(N,N-diethyl-carbamoylmethyl)-, F. 160°
-5-(N,N-diisobutyl-carbamoylmethyl)-
-5-(N-phenyl-carbamoylmethyl)-
-5-(N-o-tolyl-carbamoylmethyl)-
-5-(N-o-trifluormethylphenyl-carbamoylmethyl)-
-5-(N-o-ethoxycarbonylphenyl-carbamoylmethyl)-
-5-(N-o-chlorphenyl-carbamoylmethyl)-
-5-[N-(2,6-dimethylphenyl)-carbamoylmethyl]-
-5-[N-(2-pyridyl)-carbamoylmethyl]-
-5-(N-methyl-N-phenyl-carbamoylmethyl)-
-5-[N-methyl-N-(1,1-dimethyl-2-phenylethyl)-carbamoylmethyl]-
-5-(N,N-diphenyl-carbamoylmethyl)-
-5-(2-carbamoylethyl)-
-5-(2-N,N-dimethyl-carbamoylethyl)-
-5-(2-N-phenyl-carbamoylethyl)-
-5-[2-N-(2,6-dimethylphenyl)-carbamoylethyl]-
-5-(3-nitro-2-oxo-propyl)-
-5-(6-BOC-amino-2-oxo-hexyl)-
-5-(N-ethoxycarbonylmethyl-N-methyl-carbamoylmethyl)-
-5-(N-methylsulfonyl-carbamoylmethyl)-
-5-(N-phenylsulfonyl-carbamoylmethyl)-
-5-aziridinocarbonylmethyl-
-5-pyrrolidinocarbonylmethyl-
-5-piperidinocarbonylmethyl-, F. 221°
-5-(2-oxopyrrolidinocarbonylmethyl)-
-5-(2-oxopiperidinocarbonylmethyl)-
-5-(4-oxopiperidinocarbonylmethyl)-, F. 189°
-5-(4-o-methoxyphenyl-piperidinocarbonylmethyl)-, F. 173°
-5-[4-(2-thienyl)-piperidinocarbonylmethyl]-
-5-(4-p-methoxybenzoyl-piperidinocarbonylmethyl)-
-5-(2-ethoxycarbonyl-pyrrolidinocarbonylmethyl)-
-5-(3-ethoxycarbonyl-piperidinocarbonylmethyl)-
-5-(3-hydroxymethyl-4-p-chlorphenyl-piperidinocarbonyl-methyl)-
-5-(3-N,N-diethylcarbamoyl-piperidinocarbonylmethyl)-
-5-(3-acetamido-pyrrolidinocarbonylmethyl)-, F. 153°
-5-morpholinocarbonylmethyl-, F. 214°
-5-(3-oxo-piperazinocarbonylmethyl)-
-5-(4-methylpiperazinocarbonylmethyl)-
-5-(4-o-methoxyphenyl-piperazinocarbonylmethyl)-, F. 156°
-5-(4-o-nitrophenyl-piperazinocarbonylmethyl)-, F. 224°
-5-(3-ethoxycarbonyl-piperazinocarbonylmethyl)-
-5-(4-BOC-piperazinocarbonylmethyl)-
-5-[4-(2-pyrimidinyl)-piperazinocarbonylmethyl]-
-5-(4-p-fluorphenylsulfonyl-piperazinocarbonylmethyl)-
-5-methylthiomethyl-
-5-methylsulfinylmethyl-
-5-methylsulfonylmethyl-

-5-phenylthiomethyl-
-5-phenylsulfinylmethyl-
-5-phenylsulfonylmethyl-, F. 193°
-5-(2-thienyl)-thiomethyl-
-5-(2-pyridylaminosulfonylmethyl)-
-5-methoxysulfonylmethyl-.

(b) Analog Beispiel 7 (b) erhält man aus den unter (a) angegebenen 2'-Cyan-biphenylyl-Verbindungen die nachstehenden 2-Ethyl-4,5-dihydro-4-oxo-3-[2'-(1H-5-Tetrazolyl)-biphenylyl-4-methyl]-5-$R^3$-3H-IP:

-5-cinnamyl-
-5-(3-ethoxycarbonyl-2-phenyl-2-propen-1-yl)-
-5-(α-methoxycarbonyl-benzyl)-
-5-(2-methoxyimino-3,3-dimethyl-butyl)-
-5-(2-oxo-3-m-tolyl-5-oxazolidinyl-methyl)-
-5-carbamoylmethyl-
-5-(N-methyl-carbamoylmethyl)-
-5-(N-ethyl-carbamoylmethyl)-
-5-(N-tert.-butyl-carbamoylmethyl)-
-5-(N,N-dimethyl-carbamoylmethyl)-, F. 182°
-5-(N,N-diethyl-carbamoylmethyl)-, F. 192°
-5-(N,N-diisobutyl-carbamoylmethyl)-
-5-(N-phenyl-carbamoylmethyl)-
-5-(N-o-tolyl-carbamoylmethyl)-
-5-(N-o-trifluormethylphenyl-carbamoylmethyl)-
-5-(N-o-ethoxycarbonylphenyl-carbamoylmethyl)-
-5-(N-o-chlorphenyl-carbamoylmethyl)-
-5-[N-(2,6-dimethylphenyl)-carbamoylmethyl]-
-5-[N-(2-pyridyl)-carbamoylmethyl]-
-5-(N-methyl-N-phenyl-carbamoylmethyl)-
-5-[N-methyl-N-(1,1-dimethyl-2-phenylethyl)-carbamoylmethyl]-
-5-(N,N-diphenyl-carbamoylmethyl)-
-5-(2-carbamoylethyl)-
-5-(2-N,N-dimethyl-carbamoylethyl)-
-5-(2-N-phenyl-carbamoylethyl)-
-5-[2-N-(2,6-dimethylphenyl)-carbamoylethyl]-
-5-(3-nitro-2-oxo-propyl)-
-5-(6-BOC-amino-2-oxo-hexyl)-
-5-(N-ethoxycarbonylmethyl-N-methyl-carbamoylmethyl)-
-5-(N-methylsulfonyl-carbamoylmethyl)-
-5-(N-phenylsulfonyl-carbamoylmethyl)-
-5-aziridinocarbonylmethyl-
-5-pyrrolidinocarbonylmethyl-
-5-piperidinocarbonylmethyl-, F. 200°
-5-(2-oxopyrrolidinocarbonylmethyl)-
-5-(2-oxopiperidinocarbonylmethyl)-
-5-(4-oxopiperidinocarbonylmethyl)-
-5-(4-o-methoxyphenyl-piperidinocarbonylmethyl)
-5-[4-(2-thienyl)-piperidinocarhonylmethyl]-
-5-(4-p-methoxybenzoyl-piperidinocarbonylmethyl)-
-5-(2-ethoxycarbonyl-pyrrolidinocarbonylmethyl)-
-5-(3-ethoxycarbonyl-piperidinocarbonylmethyl)-
-5-(3-hydroxymethyl-4-p-chlorphenyl-piperidinocarbonylmethyl)-
-5-(3-N,N-diethylcarbamoyl-piperidinocarbonylmethyl)-
-5-(3-acetamido-pyrrolidinocarbonylmethyl)-, F. 187°
-5-morpholinocarbonylmethyl-, F. 196°
-5-(3-oxo-piperazinocarbonylmethyl)-
-5-(4-methylpiperazinocarbonylmethyl)-
-5-(4-o-methoxyphenyl-piperazinocarbonylmethyl)-, F. 163°
-5-(4-o-nitrophenyl-piperazinocarbonylmethyl)-, F. 223°

-5-(3-ethoxycarbonyl-piperazinocarbonylmethyl)-
-5-(4-BOC-piperazinocarbonylmethyl)-
-5-[4-(2-pyrimidinyl)-piperazinocarbonylmethyl]-
-5-(4-p-fluorophenylsulfonyl-piperazinocarbonylmethyl)-
-5-methylthiomethyl-
-5-methylsulfinylmethyl-
-5-methylsulfonylmethyl-
-5-phenylthiomethyl-
-5-phenylsulfinylmethyl-
-5-phenylsulfonylmethyl-, F. 168°
-5-(2-thienyl)-thiomethyl-
-5-(2-pyridylaminosulfonylmethyl)-
-5-methoxysulfonylmethyl-.

Beispiel 9

(a) Analog Beispiel 7 (a) erhält man aus 2-Cyclopropyl-3-(2'-cyan-biphenylyl-4-methyl)-4,5-dihydro-4-oxo-3H-IP (F. 183°; erhältlich aus 2-Cyclopropyl-4,5-dihydro-4-oxo-1(oder 3)H-IP mit 4'-Brommethyl-2-cyan-biphenyl) und den in Beispiel 7 (a) angegebenen Verbindungen der Formel E-$R^3$ die nachstehenden 2-Cyclopropyl-3-(2'-cyan-biphenylyl-4-methyl)-4,5-dihydro-4-oxo-5-$R^3$-3H-IP:
-5-cinnamyl-
-5-(3-ethoxycarbonyl-2-phenyl-2-propen-1-yl)-
-5-($\alpha$-methoxycarbonyl-benzyl)-
-5-(2-methoxyimino-3,3-dimethyl-butyl)-
-5-(2-oxo-3-m-tolyl-5-oxazolidinyl-methyl)-
-5-carbamoylmethyl-
-5-(N-methyl-carbamoylmethyl)-
-5-(N-ethyl-carbamoylmethyl)-
-5-(N-tert.-butyl-carbamoylmethyl)-
-5-(N,N-dimethyl-carbamoylmethyl)-, Rf 0,16 (Ethylacetat/Methanol 9:1)
-5-(N,N-diethyl-carbamoylmethyl)-
-5-(N,N-diisobutyl-carbamoylmethyl)-
-5-(N-phenyl-carbamoylmethyl)-
-5-(N-o-tolyl-carbamoylmethyl)-
-5-(N-o-trifluormethylphenyl-carbamoylmethyl)-
-5-(N-o-ethoxycarbonylphenyl-carbamoylmethyl)-
-5-(N-o-chlorphenyl-carbamoylmethyl)-
-5-[N-(2,6-dimethylphenyl)-carbamoylmethyl]-
-5-[N-(2-pyridyl)-carbamoylmethyl]-
-5-(N-methyl-N-phenyl-carbamoylmethyl)-
-5-[N-methyl-N-(1,1-dimethyl-2-phenylethyl) carbamoylmethyl]-
-5-(N,N-diphenyl-carbamoylmethyl)-, Rf 0,39 (Ethylacetat/Methanol 9:1)
-5-(2-carbamoylethyl)-
-5-(2-N,N-dimethyl-carbamoylethyl)-
-5-(2-N-phenyl-carbamoylethyl)-
-5-[2-N-(2,6-dimethylphenyl)-carbamoylethyl]-
-5-(3-nitro-2-oxo-propyl)-
-5-(6-BOC-amino-2-oxo-hexyl)-
-5-(N-ethoxycarbonylmethyl-N-methyl-carbamoylmethyl)-
-5-(N-methylsulfonyl-carbamoylmethyl)-
-5-(N-phenylsulfonyl-carbamoylmethyl)-
-5-aziridinocarbonylmethyl-
-5-pyrrolidinocarbonylmethyl-
-5-piperidinocarbonylmethyl-
-5-(2-oxopyrrolidinocarbonylmethyl)-
-5-(2-oxopiperidinocarbonylmethyl)-
-5-(4-oxopiperidinocarbonylmethyl)-
-5-(4-o-methoxyphenyl-piperidinocarbonylmethyl)

-5-[4-(2-thienyl)-piperidinocarbonylmethyl]-
-5-(4-p-methoxybenzoyl-piperidinocarbonylmethyl)-
-5-(2-ethoxycarbonyl-pyrrolidinocarbonylmethyl)-
-5-(3-ethoxycarbonyl-piperidinocarbonylmethyl)-
-5-(3-hydroxymethyl-4-p-chlorphenyl-piperidinocarbonylmethyl)-
-5-(3-N,N-diethylcarbamoyl-piperidinocarbonylmethyl)-
-5-(3-acetamido-pyrrolidinocarbonylmethyl)-
-5-morpholinocarbonylmethyl-
-5-(3-oxo-piperazinocarbonylmethyl)-
-5-(4-methylpiperazinocarbonylmethyl)-
-5-(4-o-methoxyphenyl-piperazinocarbonylmethyl)-
-5-(4-o-nitrophenyl-piperazinocarbonylmethyl)-
-5-(3-ethoxycarbonyl-piperazinocarbonylmethyl)-
-5-(4-BOC-piperazinocarbonylmethyl)-
-5-[4-(2-pyrimidinyl)-piperazinocarbonylmethyl]-
-5-(4-p-fluorphenylsulfonyl-piperazinocarbonylmethyl)-
-5-methylthiomethyl-
-5-methylsulfinylmethyl-
-5-methylsulfonylmethyl-
-5-phenylthiomethyl-
-5-phenylsulfinylmethyl-
-5-phenylsulfonylmethyl-
-5-(2-thienyl)-thiomethyl-
-5-(2-pyridylaminosulfonylmethyl)-
-5-methoxysulfonylmethyl-.

(b) Analog Beispiel 7 (b) erhält man aus den unter (a) angegebenen 2'-Cyan-biphenylyl-Verbindungen die nachstehenden 2-Cyclopropyl-4,5-dihydro-4-oxo-3-[2'-(1H-5-tetrazolyl)-biphenylyl-4-methyl]-5-$R^3$-3H-IP:

-5-cinnamyl-
-5-(3-ethoxycarbonyl-2-phenyl-2-propen-1-yl)-
-5-(α-methoxycarbonyl-benzyl)-
-5-(2-methoxyimino-3,3-dimethyl-butyl)-
-5-(2-oxo-3-m-tolyl-5-oxazolidinyl-methyl)-
-5-carbamoylmethyl-
-5-(N-methyl-carbamoylmethyl)-
-5-(N-ethyl-carbamoylmethyl)-
-5-(N-tert.-butyl-carbamoylmethyl)-
-5-(N,N-dimethyl-carbamoylmethyl)-, F. 231°
-5-(N,N-diethyl-carbamoylmethyl)-
-5-(N,N-diisobutyl-carbamoylmethyl)-
-5-(N-phenyl-carbamoylmethyl)-
-5-(N-o-tolyl-carbamoylmethyl)-
-5-(N-o-trifluormethylphenyl-carbamoylmethyl)-
-5-(N-o-ethoxycarbonylphenyl-carbamoylmethyl)-
-5-(N-o-chlorphenyl-carbamoylmethyl)-
-5-[N-(2,6-dimethylphenyl)-carbamoylmethyl]-
-5-[N-(2-pyridyl)-carbamoylmethyl]-
-5-(N-methyl-N-phenyl-carbamoylmethyl)-
-5-[N-methyl-N-(1,1-dimethyl-2-phenylethyl)-carbamoylmethyl]-
-5-(N,N-diphenyl-carbamoylmethyl)-, F. 204°
-5-(2-carbamoylethyl)-
-5-(2-N,N-dimethyl-carbamoylethyl)-
-5-(2-N-phenyl-carbamoylethyl)-
-5-[2-N-(2,6-dimethylphenyl)-carbamoylethy]-
-5-(3-nitro-2-oxo-propyl)-
-5-(6-BOC-mino-2-oxo-hexyl)-
-5-(N-ethoxycarbonylmethyl-N-methyl-carbmoylmethyl)-
-5-(N-methylsulfonyl-carbamoylmethyl)-

-5-(N-phenylsulfonyl-carbamoylmethyl)-
-5-aziridinocarbonylmethyl-
-5-pyrrolidinocarbonylmethyl-
-5-piperidinocarbonylmethyl-
-5-(2-oxopyrrolidinocarbonylmethyl)-
-5-(2-oxopiperidinocarbonylmethyl)-
-5-(4-oxopiperidinocarbonylmethyl)-
-5-(4-o-methoxyphenyl-piperidinocarbonylmethyl)
-5-[4-(2-thienyl)-piperidinocarbonylmethy]-
-5-(4-p-methoxybenzoyl-piperidinocarbonylmethyl)-
-5-(2-ethoxycarbonyl-pyrrolidinocarbonylmethyl)-
-5-(3-ethoxycarbonyl-piperidinocarbonylmethyl)-
-5-(3-hydroxymethyl-4-p-chlorphenyl-piperidinocarbonyl-methyl)-
-5-(3,N,N-diethylcarbamoyl-piperidinocarbonylmethyl)-
-5-(3-acetamido-pyrrolidinocarbonylmethyl)-
-5-morpholinocarbonylmethyl-
-5-(3-oxo-piperazinocarbonylmethyl)-
-5-(4-methylpiperazinocarbonylmethyl)-
-5-(4-o-methoxyphenyl-piperazinocarbonylmethyl)-
-5-(4-o-nitrophenyl-piperazinocarbonylmethyl)-
-5-(3-ethoxycarbonyl-piperazinocarbonylmethyl)-
-5-(4-BOC-piperazinocarbonylmethyl)-
-5-[4-(2-pyrimidinyl)-piperazinocarbonylmethyl]-
-5-(4-p-fluorphenylsulfonyl-piperazinocarbonylmethyl)-
-5-methylthiomethyl-
-5-methylsulfinylmethyl-
-5-methylsulfonylmethyl-
-5-phenylthiomethyl-
-5-phenylsulfinylmethyl-
-5-phenylsulfonylmethyl-
-5-(2-thienyl)-thiomethyl-
-5-(2-pyridylaminosulfonylmethyl)-
-5-methoxysulfonylmethyl-.

Beispiel 10

(a) Analog Beispiel 7 (a) erhält man aus 2-Butyl-4,5-dihydro-4-oxo-3-[2'-(2-triphenylmethyl-2H-5-tetrazolyl)-biphenylyl-4-methyl]-3H-IP mit N,N-Dimethylchloracetamid das 2-Butyl-4,5-dihydro-5-(N,N-dimethylcarbamoylmethyl)-4-oxo-3-[2'-(2-triphenylmethyl-2H-5-tetrazolyl)-biphenylyl-4-methyl]-3H-IP, F. 187°.

Analog erhält man mit den in Beispiel 7 (a) angegebenen Verbindungen der Formel E-$R^3$ die nachstehenden 2-Butyl-4,5-dihydro-4-oxo-3-[2'-(2-triphenylmethyl-2H-5-tetrazolyl)-biphenylyl-4-methy]-5-$R^3$-3H-IP:
-5-cinnamyl-, F. 89°
-5-(3)-ethoxycarbonyl-2-phenyl-2-propen-1-yl)-, F. 89°
-5-($\alpha$-methoxycarbonyl-benzyl)-, F. 94°
-5-(2-methoxyimino-3,3-dimethyl-butyl)-
-5-(2-oxo-3-m-tolyl-5-oxzaolidinyl-methyl)-, F. 107°
-5-carbamoylmethyl-
-5-(N-methyl-carbmoylmethyl)-
-5-(N-ethyl-carbmoylmethyl)-
-5-(N-tert.-butyl-carbamoylmethyl)-
-5-(N,N-diethyl-carbamoylmethyl)-
-5-(N,N-diisobutyl-carbamoylmethyl)-
-5-(N-phenyl-carbamoylmethyl)-
-5-(N-o-tolyl-carbamoylmethyl)-
-5-(N-o-trifluomethylphenyl-carbmoylmethyl)-
-5-(N-o-ethoxycarbonylphenyl-carbamoylmethyl)-
-5-(N-o-chlorphenyl-carbamoylmethyl)-

-5-[N-(2,6-dimethylphenyl)-carbamoylmethyl]-
-5-[N-(2-pyridyl)-carbamoylmethyl]-
-5-(N-methyl-N-phenyl-carbamoylmethyl)-
-5-[N-methyl-N-(1,1-dimethyl-2-phenylethyl)-carbamoylmethyl]-
-5-(N,N-diphenyl-carbamoylmethyl)-
-5-(2-carbamoylethyl)-
-5-(2-N,N-dimethyl-carbamoylethyl)-
-5-(2-N-phenyl-carbamoylethyl)-
-5-[2-N-(2,6-dimethylphenyl)-carbamoylethyl]-
-5-(3-nitro-2-oxo-propyl)-
-5-(6-BOC-amino-2-oxohexyl)-
-5-(N-ethoxycarbonylmethyl-N-methyl-carbamoylmethyl)-
-5-(N-methylsulfonyl-carbamoylmethyl)-
-5-(N-phenylsulfonyl-carbamoylmethyl)-
-5-aziridinocarbonylmethyl-
-5-pyrrolidinocarbonylmethyl-
-5-piperidinocarbonylmethyl-
-5-(2-oxopyrrolidinocarbonylmethyl)-
-5-(2-oxopiperidinocarbonylmethyl)-
-5-(4-oxopiperidinocarbonylmethyl)-
-5-(4-o-methoxyphenyl-piperidinocarbonylmethyl)
-5-[4-(2-thienyl)-piperidinocarbonylmethyl]-
-5-(4-p-methoxybenzoyl-piperidinocarbonylmethyl)-
-5-(2-ethoxycarbonyl-pyrrolidinocarbonylmethyl)-
-5-(3-ethoxycarbonyl-piperidinocarbonylmethyl)-
-5-(3-hydroxymethyl-4-p-chlorphenyl-piperidinocarbonylmethyl)-
-5-(3-N,N-diethylcarbamoyl-piperidinocarbonylmethyl)-
-5-(3-acetamido-pyrrolidinocarbonylmethyl)-
-5-morpholinocarbonylmethyl-
-5-(3-oxo-piperazinocarbonylmethyl)-
-5-(4-methylpiperazinocarbonylmethyl)-
-5-(4-o-methoxyphenyl-piperazinocarbonylmethyl)-
-5-(4-o-nitrophenyl-piperazinocarbonylmethyl)-
-5-(3-ethoxycarbonyl-piperazinocarbonylmethyl)-
-5-(4-BOC-piperazinocarbonylmethyl)-
-5-[4-(2-pyrimidinyl)-piperazinocarbonylmethyl]-
-5-(4-p-fluorphenylsulfonyl-piperazinocarbonylmethyl)-
-5-methylthiomethyl-
-5-methylsulfinylmethyl-
-5-methylsulfonylmethyl-
-5-phenylthiomethyl-
-5-phenylsulfinylmethyl-
-5-phenylsulfonylmethyl-
-5-(2-thienyl)-thiomethyl-
-5-(2-pyridyIminosulfonylmethyl)-
-5-methoxysulfonylmethyl-
sowie die nachstehenden 2-Ethyl-4,5-dihydro-4-oxo-3-[2'-(2-triphenylmethyl-2H-5-tetrazolyl)-biphenylyl-4-methyl]-5-$R^3$-3H-IP:
-5-cinnamyl-
-5-(3-ethoxycarbonyl-2-phenyl-2-propen-1-yl)-
-5-($\alpha$-methoxycarbonyl-benzyl)-
-5-(2-methoxyimino-3,3-dimethyl-butyl)-
-5-(2-oxo-3-m-tolyl-5-oxazolidinyl-methyl)-
-5-carbamoylmethyl-
-5-(N-methyl-carbamoylmethyl)-
-5-(N-ethyl-carbamoylmethyl)-
-5-(N-tert.-butyl-carbamoylmethyl)-
-5-(N,N-dimethyl-carbamoylmethyl)-

-5-(N,N-diethyl-carbamoylmethyl)-
-5-(N,N-diisobutyl-carbamoylmethyl)-
-5-(N-phenyl-carbamoylmethyl)-
-5-(N-o-tolyl-carbamoylmethyl)-
-5-(N-o-trifluormethylphenyl-carbamoylmethyl)-
-5-(N-o-ethoxycarbonylphenyl-carbamoylmethyl)-
-5-(N-o-chlorphenyl-carbamoylmethyl)-
-5-[N-(2,6-dimethylphenyl)-carbamoylmethyl]-
-5-[N-(2-pyridyl)-carbamoylmethyl]-
-5-(N-methyl-N-phenyl-carbamoylmethyl)-
-5-[N-methyl-N-(1,1-dimethyl-2-phenylethyl)-carbamoylmethyl]-
-5-(N,N-diphenyl-carbamoylmethyl)-
-5-(2-carbamoylethyl)-
-5-(2-N,N-dimethyl-carbamoylethyl)-
-5-(2-N-phenyl-carbamoylethyl)-
-5-[2-N-(2,6-dimethylphenyl)-carbamoylethyl]-
-5-(3-nitro-2-oxo-propyl)-
-5-(6-BOC-amino-2-oxo-hexyl)-
-5-(N-ethoxycarbonylmethyl-N-methyl-carbamoylmethyl)-
-5-(N-methylsulfonyl-carbamoylmethyl)-
-5-(N-phenylsulfonyl-carbamoylmethyl)-
-5-aziridinocarbonylmethyl-
-5-pyrrolidinocarbonylmethyl-
-5-piperidinocarbonylmethyl-
-5-(2-oxopyrrolidinocarbonylmethyl)-
-5-(2-oxopiperidinocarbonylmethyl)-
-5-(4-oxopiperidinocarbonylmethyl)-
-5-(4-o-methoxyphenyl-piperidinocarbonylmethyl)
-5-[4-(2-thienyl)-piperidinocarbonylmethyl]-
-5-(4-p-methoxybenzoyl-piperidinocarbonylmethyl)-
-5-(2-ethoxycarbonyl-pyrrolidinocarbonylmethyl)-
-5-(3-ethoxycarbonyl-piperidinocarbonylmethyl)-
-5-(3-hydroxymethyl-4-p-chlorphenyl-piperidinocarbonyl-methyl)-
-5-(3-N,N-diethylcarbamoyl-piperidinocarbonylmethyl)-
-5-(3-acetamido-pyrrolidinocarbonylmethyl)-
-5-morpholinocarbonylmethyl-
-5-(3-oxo-piperazinocarbonylmethyl)-
-5-(4-methylpiperazinocarbonylmethyl)-
-5-(4-o-methoxyphenyl-piperazinocarbonylmethyl)-
-5-(4-o-nitrophenyl-piperazinocarbonylmethyl)-
-5-(3)-ethoxycarbonyl-piperazinocarbonylmethyl)-
-5-(4-BOC-piperazinocarbonylmethyl)-
-5-[4-(2-pyrimidinyl)-piperazinocarbonylmethyl]-
-5-(4-p-fluorphenylsulfonyl-piperazinocarbonylmethyl)-
-5-methylthiomethyl-
-5-methylsulfinylmethyl-
-5-methylsulfonylmethyl-
-5-phenylthiomethyl-
-5-phenylsulfinylmethyl-
-5-phenylsulfonylmethyl-
-5-(2-thienyl)-thiomethyl-
-5-(2-pyridylaminosulfonylmethyl)-
-5-methoxysulfonylmethyl-.

(b) Das nach (a) erhaltene Produkt (1 g) wird in 60 ml 4n HCl in Dioxan gelöst und 16 Stunden bei 20° gerührt. Man dampft ein, arbeitet wie üblich auf und erhält 2-Butyl-4,5-dihydro-5-(N,N-dimethylcarbamoylmethyl)-4-oxo-3-[2'-(1H-5-tetrazolyl)-biphenylyl-4-methyl]-3H-IP, F. 258°.

Analog erhält man aus den unter (a) angegebenen entsprechenden 2-Triphenylmethyl-2H-5-tetrazolyl-verbindungen die in den Beispielen 7 (b) und 8 (b) angegebenen 1H-5-Tetrazolyl-verbindun-

gen.

Beispiel 11

Analog Beispiel 7 (a) erhält man aus 2-Butyl-3-(p-2-cyan-2-phenyl-vinyl-benzyl)-4,5-dihydro-4-oxo-3H-IP (F. 160°; erhältlich aus 2-Butyl-4,5-dihydro-4-oxo-1 (oder 3)H-IP und 3-p-Brommethylphenyl-2-phenyl-acrylnitril) mit N,N-Dimethylchloracetamid das 2-Butyl-3-(p-2-cyan-2-phenyl-vinyl-benzyl)-4,5-dihydro-5-(N,N-dimethylcarbamoylmethyl)-4-oxo-3H-IP.

Beispiel 12

Zu einer Lösung von 0,44 g 2-Butyl-3-(2'-cyan-biphenylyl-4-methyl)-4,5-dihydro-4-oxo-3H-IP-5-essigsäure ["B"; F. 222°; erhältlich durch Reaktion von 2-Butyl-3-(2'-cyanbiphenylyl-4-methyl)-4,5-dihydro-4-oxo-3H-IP mit Bromessigsäureethylester zum 5-Ethoxycarbonylmethyl-derivat (F. 152°) und anschließende Verseifung] in 14 ml THF gibt man 210 mg DCCI, rührt 10 Min. bei 20°, setzt 72 mg Pyrrolidin zu und rührt weitere 18 Std. bei 20°. Man filtriert, arbeitet das Filtrat wie üblich auf, chromatographiert das Rohprodukt an Kieselgel (Ethylacetat/Methanol 80:20) und erhält 2-Butyl-3-(2'-cyan-biphenylyl-4-methyl)-4,5-dihydro-4-oxo-5-pyrrolidinocarbonylmethyl-3H-IP, F. 166°.

Beispiel 13

Zu einer Lösung von 4,4 g "B" und 2,44 g 1-p-Fluorphenylsulfonylpiperazin in 90 ml DMF gibt man nacheinander 1,94 g DAPECI, 1,36 g 1-Hydroxy-benzotriazol und 1,1 ml N-Methylmorpholin, rührt das Gemisch 5 Std. bei 20°, fällt mit Wasser das Produkt aus, filtriert ab, wäscht mit Wasser, trocknet und erhält 2-Butyl-3-(2'-cyan-biphenylyl-4-methyl)-4,5-dihydro-4-oxo-5-(4-p-fluorphenylsulfonyl-piperazino-carbonylmethyl-3H-IP, F. 207°.

Beispiel 14

Eine Lösung von 4,4 g "B" in 20 ml THF wird unter Rühren zu einer Lösung von 1,6 g 1,1'-Carbonyldiimidazol in 20 ml THF zugetropft und anschließend 30 Min. erhitzt. Nach dem Abkühlen gibt man 1,6 g Benzolsulfonamid hinzu, rührt 10 Min., tropft eine Lösung von 1,48 g 1,8-Diazabicyclo[5,4,0]undec-7-en in 10 ml THF hinzu, rührt 18 Std. bei 20°, arbeitet wie üblich auf (1n Salzsäure/Dichlormethan) und erhält 2-Butyl-3-(2'-cyan-biphenylyl-4-methyl)-4,5-dihydro-4-oxo-5-(N-phenylsulfonyl-carbamoylmethyl)-3H-IP, F. 193°.

Beispiel 15

Analog Beispiel 12 erhält man aus 2-Ethyl-3-(2'-cyan-biphenylyl-4-methyl)-4,5-dihydro-4-oxo-3H-IP-5-essigsäure [F. 221°; erhältlich durch Reaktion von 2-Ethyl-3-(2'-cyan-biphenylyl-4-methyl)-4,5-dihydro-4-oxo-3H-IP mit Bromessigsäureethylester zu 2-Ethyl-3-(2'-cyan-biphenylyl-4-methyl)-4,5-dihydro-4-oxo-5-ethoxycarbonylmethyl-3H-IP (F. 143°) und anschließende Verseifung] und Diethylamin in Gegenwart von DCCI das 2-Ethyl-3-(2'-cyan-biphenylyl-4-methyl)-4,5-dihydro-4-oxo-5-(N,N-diethyl-carbamoylmethyl)-3H-IP, F. 160°.

Beispiel 16

Analog Beispiel 1(b) erhält man durch Verseifung der entsprechenden in Beispiel 7(b) angegebenen Ethylester die nachstehenden 2-Butyl-4,5-dihydro-4-oxo-3-[2'-(1H-5-tetrazolyl)-biphenylyl-4-methyl]-5-$R^3$-3H-IP:
-5-(3-carboxy-2-phenyl-2-propen-1-yl)-
-5-(N-o-carboxyphenyl-carbamoylmethyl)-.

Beispiel 17

Eine Lösung von 1 g 2-Butyl-4,5-dihydro-5-(3-nitro-2-oxo-propyl)-4-oxo-3-[2'-(1H-5-tetrazolyl)-biphenylyl-4-methyl]-3H-IP in 20 ml Methanol wird an 0,3 g 5%iger Pd-Kohle bei 20° und Normaldruck bis zur Aufnahme der berechneten $H_2$-Menge hydriert. Man filtriert den Katalysator ab, dampft ein und erhält 5-(3-Amino-2-oxo-propyl)-2-butyl-4,5-dihydro-4-oxo-3-[2'-(1H-5-tetrazolyl)-biphenylyl-4-methyl]-3H-IP.

Beispiel 18

Eine Lösung von 1 g 5-(6-BOC-amino-2-oxo-hexyl)-2-butyl-4,5-dihydro-4-oxo-3-[2'-(1H-5-tetrazolyl)-biphenylyl-4-methyl]-3H-IP in 20 ml Dichlormethan und 20 ml Trifluoressigsäure wird 1 Std. bei 20° gerührt, eingedampft und wie üblich aufgearbeitet. Man erhält 5-(6-Amino-2-oxo-hexyl)-2-butyl-4,5-dihydro-4-oxo-3-[2'-(1H-5-tetrazolyl)-biphenylyl-4-methyl]-3H-IP.

Analog erhält man aus 2-Ethyl- bzw. aus 2-Butyl-4,5-dihydro-4-oxo-3-[2'-(1H-5-tetrazolyl)-biphenylyl-4-methyl]-5-(4-BOC-piperazino-carbonylmethyl)-3H-IP das 2-Ethyl- bzw. das 2-Butyl-4,5-dihydro-4-oxo-3-[2'-(1H-5-tetrazolyl)-biphenylyl-4-methyl]-5-(4-piperazinocarbonylmethyl)-3H-IP.

Beispiel 19

Ein Gemisch von 7,67 g 2-Butyl-4,5-dihydro-5-(3,3-dimethyl-2-oxo-butyl)-3-[2'-(2-triphenylmethyl-2H-5-tetrazolyl)-biphenylyl-4-methyl]-3H-IP, 1,67 g o-Methyl-hydroxylamin-hydrochlorid, 200 ml Methanol und 3,2 g Pyridin wird 72 Std. bei 20° gerührt. Intermediär entsteht 2-Butyl-4,5-dihydro-5-(3,3-dimethyl-2-oxo-butyl)-3-[2'-(1H-5-tetrazolyl)-biphenylyl-4-methyl]-3H-IP, das nicht isoliert wird. Nach üblicher Aufarbeitung (Chromatographie an Kieselgel mit Ethylacetat/Methanol) erhält man 2-Butyl-4,5-dihydro-5-(3,3-dimethyl-2-methoxyimino-butyl)-3-[2'-(1H-5-tetrazolyl)-biphenylyl-4-methyl]-3H-IP, F. 83°.

Die nachstehenden Beispiele betreffen pharmazeutische Zubereitungen, die Wirkstoffe der Formel I oder ihre Salze enthalten.

Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

Beispiel C: Weichgelatine-Kapseln

Übliche Weichgelatine-Kapseln werden mit einem Gemisch aus jeweils 50 mg Wirkstoff und 250 mg Olivenöl gefüllt.

Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

Beispiel E: Wässerige Suspension für orale Applikation

Eine wässerige Suspension des Wirkstoffs wird in üblicher Weise hergestellt. Die Einheitsdosis (5 ml) enthält 100 mg Wirkstoff, 100 mg Na-Carboxymethylcellulose, 5 mg Na-Benzoat und 100 mg Sorbit.

## Patentansprüche

**1.** Imidazopyridinderivate der Formel I

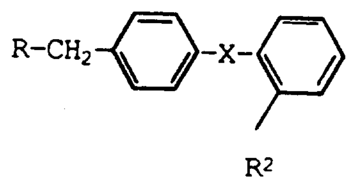

I

worin

R

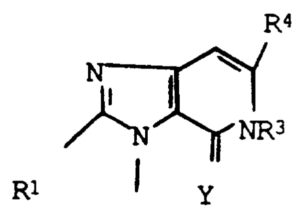

| | |
|---|---|
| $R^1$ | A, Alkenyl oder Alkinyl mit jeweils bis zu 6 C-Atomen, $C_3$-$C_7$-Cycloalkyl-$C_kH_{2k}$- oder $C_1$-$C_6$-Alkyl, worin eine $CH_2$-Gruppe durch O oder S ersetzt ist, |
| $R^2$ | H, COOH, COOA, CN, $NO_2$, $NHCOR^5$, $NHSO_2R^5$ oder 1H-5-Tetrazolyl, |
| $R^3$ | ein- oder mehrfach durch COOH, COOA, CN, $NO_2$, $NR^6R^7$, $NHCOR^8$, $NHSO_2R^8$, Hal und/oder Ar substituiertes Alkenyl mit 2-6 C-Atomen im "alkenyl"-Teil, $-C_nH_{2n}-R^9$ oder $-CHR^{10}-C_kH_{2K}-R^{11}$, |
| $R^4$ | H oder Hal, |
| $R^5$ und $R^8$ | jeweils Alkyl mit 1-5 C-Atomen, worin auch ein oder mehrere H-Atom(e) durch F ersetzt sein können, |
| $R^6$ und $R^7$ | jeweils H, A, Alkenyl oder Alkinyl mit jeweils bis zu 6 C-Atomen, Ar, $ArC_nH_{2n}$- oder $Het^2$, |
| $R^6$ auch | $-CH_2COOA$, $-SO_2$-A oder $-SO_2$-Ar, |
| $R^6$ und $R^7$ | zusammen auch eine Alkylenkette mit 2-5 C-Atomen, die ein- oder mehrfach durch Carbonylsauerstoff, Ar, $Het^2$, -CO-Ar, -COOA, $-CO-N(A)_2$, $-CH_2OH$, $-SO_2$-Ar und/oder -NH-CO-A substituiert und/oder durch O oder durch $-NR^{16}$- unterbrochen sein kann, |
| $R^9$ | Cycloalkyl mit 3-8 C-Atomen, $Het^1$, $-CO-NR^6R^7$, $-CO-R^{14}$, $-C(=NR^{12})$-A, $-C(=NR^{12})-Het^2$, $-S(O)_m$-A, $-S(O)_m$-Ar, $-S(O)_m-Het^2$, $-SO_2-NH-Het^2$ oder $-SO_2-OR^{15}$, |
| $R^{10}$ | COOH, COOA, CN, $NO_2$, $NHCOR^{11}$, $NHSO_2R^{11}$ oder 1H-5-Tetrazolyl, |
| $R^{11}$ | Ar oder Cycloalkyl mit 3-8 C-Atomen, |
| $R^{12}$ | H, OH, CN, $R^{13}$, $OR^{13}$ oder OAr, |
| $R^{13}$ | A, Alkenyl oder Alkinyl mit jeweils bis zu 6 C-Atomen, |
| $R^{14}$ | $-NH-CHR^{15}-COOH$, $-NH-CHR^{15}-COOA$, $-CH_2S(O)_m$-Ar, $-CH_2$-COOA, $-C_nH_{2n}-NO_2$, $-C_nH_{2n}-NR^6R^7$ oder $-C_nH_{2n}$-NHCOOA, |
| $R^{15}$ | H oder A, |
| $R^{16}$ | H, A, Ar, COOA, $Het^2$ oder $-SO_2$-Ar, |
| X | fehlt, -NH-CO-, -CO-NH-, -O-CH(COOH)-, -NH-CH(COOH)-, -NA-CH(COOH), -CH=C-(COOH), -CH=C(CN) oder -CH=C(1H-5-tetrazolyl)-, |
| Y | O oder S, |
| A | Alkyl mit 1-6 C-Atomen, |
| Ar | eine unsubstituierte oder eine durch $R^5$, $OR^5$, COOH, COOA, CN, $NO_2$, $NH_2$, |

NHCOR$^5$, $NHSO_2R^5$, Hal oder 1H-5-Tetrazolyl mono- oder disubstituierte Phenylgruppe,

Het$^1$ einen fünf- oder sechsgliedrigen gesättigten heterocyclischen Rest mit 1 bis 3 N-, O- und/oder S-Atomen, der einfach durch Carbonylsauerstoff oder $=NR^{12}$ und/oder dessen Ring-N-Atom(e) jeweils durch A oder Ar substituiert sein kann (können),

Het$^2$ einen fünf- oder sechsgliedrigen heteroaromatischen Rest mit 1 bis 3 N-, O- und/oder S-Atomen, der auch mit einem Benzol- oder Pyridinring kondensiert sein kann und/oder ein- oder mehrfach durch A substituiert sein kann,

Hal F, Cl, Br oder I,

k 0, 1, 2, 3 oder 4,

m 0, 1 oder 2 und

n 1, 2, 3, 4, 5 oder 6 bedeuten,

sowie ihre Salze.

**2.**

a) 2-Butyl-5-cinnamyl-4,5-dihydro-4-oxo-3-[2'-(1H-5-tetrazolyl)-biphenylyl-4-methyl]-3H-imidazo[4,5-c]pyridin;

b) 2-Butyl-5-(3-ethoxycarbonyl-2-phenyl-2-propen-1-yl)-4,5-dihydro-4-oxo-3-[2'-(1H-5-tetrazolyl)-biphenylyl-4-methyl]-3H-imidazo[4,5-c]pyridin;

c) 2-Butyl-4,5-dihydro-5-(N,N-dimethylcarbamoyl-methyl)-4-oxo-3-[2'-(1H-5-tetrazolyl)-biphenylyl-4-methyl]-3H-imidazo[4,5-c]pyridin;

d) 2-Butyl-4,5-dihydro-5-α-methoxycarbonyl-benzyl-4-oxo-3-[2'-(1H-5-tetrazolyl)-biphenylyl-4-methyl]-3H-imidazo[4,5-c]pyridin;

e) 2-Butyl-4,5-dihydro-5-(2-methoxyimino-3,3-dimethylbutyl-4-oxo-3-[2'-(1H-5-tetrazolyl)-biphenylyl-4-methyl]-3H-imidazo[4,5-c]pyridin;

f) 2-Butyl-4,5-dihydro-4-oxo-5-(2-oxo-3-m-tolyl-5-oxazolidinyl-methyl)-3-[2'-(1H-5-tetrazolyl)-biphenylyl-4-methyl]-3H-imidazo[4,5-c]pyridin.

**3.** Verfahren zur Herstellung von Imidazopyridinen der Formel I nach Anspruch 1 sowie ihrer Salze, dadurch gekennzeichnet, daß man

(a) eine Verbindung der Formel II

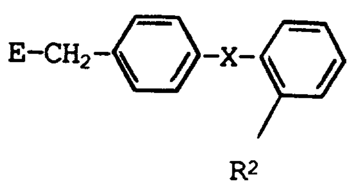

II

worin

E Cl, Br, I oder eine freie oder reaktionsfähig funktionell angewandelte OH-Gruppe bedeutet und

$R^2$ und X die in Anspruch 1 angegebene Bedeutung haben,

mit einer Verbindung der Formel III

H-R (III)

worin

R die in Anspruch 1 angegebene Bedeutung hat,

umsetzt

oder

(b) eine Verbindung der Formel IV

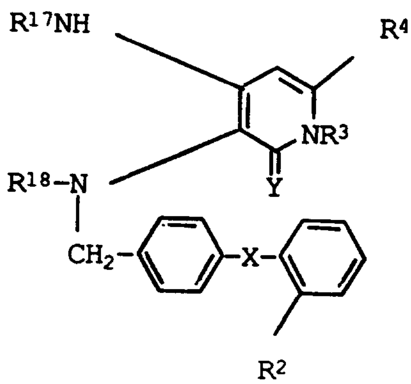

IV

worin

$R^{17}$ $R^1$-CO oder H und

$R^{18}$ H (falls $R^{17}$ $R^1$-CO ist) oder $R^1$-CO (falls $R^{17}$ H ist)

bedeuten

und

$R^1$, $R^2$, $R^3$, $R^4$, X und Y die in Anspruch 1 angegebenen Bedeutungen haben,

mit einem cyclisierenden Mittel behandelt,

oder

(c) zur Herstellung einer Verbindung der Formel I, worin X -NH-CO- oder -CO-NH- bedeutet, eine Verbindung der Formel V

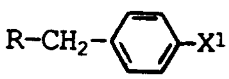

V

worin

$X^1$ $NH_2$ oder COOH bedeutet und

R die in Anspruch 1 angegebene Bedeutung hat

oder ein reaktionsfähiges Derivat dieser Verbindung mit einer Verbindung der Formel VI

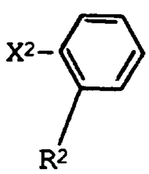

VI

worin

$X^2$ COOH (falls $X^1$ $NH_2$ ist) oder $NH_2$ (falls $X^1$ COOH ist) bedeutet und

$R^2$ die in Anspruch 1 angegebene Bedeutung hat,

oder mit einem reaktionsfähigen Derivat dieser Verbindung umsetzt oder

(d) eine Verbindung der Formel VII

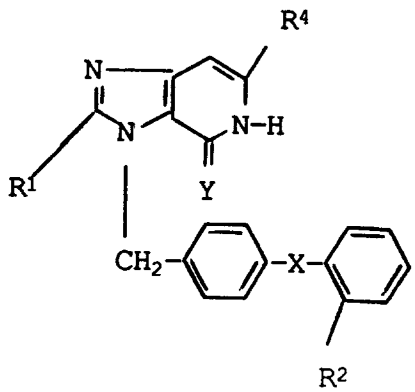

VII

worin
$R^1$, $R^2$, $R^4$, X und Y die in Anspruch 1 angegebenen Bedeutungen haben, mit einer Verbindung der Formel VIII

$E\text{-}R^3$ VIII

worin
$R^3$ und E die in Anspruch 1 bzw. 3 angegebenen Bedeutungen haben, umsetzt oder

(e) zur Herstellung einer Verbindung der Formel I, die eine $-C(=NR^{12})$-Gruppe enthält, eine entsprechende Carbonylverbindung mit einer Verbindung der Formel $H_2N\text{-}R^{12}$, worin $R^{12}$ die in Anspruch 1 angegebene Bedeutung hat, behandelt oder

(f) zur Herstellung einer Verbindung, worin $R^3$ $-C_nH_{2n}\text{-}CO\text{-}R^{19}$ und $R^{19}$ $-NR^6R^7$ oder $-NH\text{-}CHR^{15}\text{-}COOA$ bedeutet, eine Carbonsäure, die der Formel I entspricht, aber an Stelle des Restes $R^3$ eine $-C_nH_{2n}\text{-}COOH$-Gruppe enthält (oder eines ihrer funktionellen Derivate) mit einer Verbindung der Formel $H\text{-}R^{19}$ umsetzt oder

(g) eine Verbindung der Formel I aus einem ihrer funktionellen Derivate durch Behandeln mit einem solvolysierenden oder hydrogenolysierenden Mittel in Freiheit setzt,

und/oder daß man in einer Verbindung der Formel I einen oder mehrere Rest(e) R und/oder $R^2$ in einen oder mehrere andere Reste R und/oder $R^2$ umwandelt und/oder eine Base oder Säure der Formel I in eines ihrer Salze umwandelt.

**4.** Verfahren zur Herstellung pharmazeutischer Zubereitungen, dadurch gekennzeichnet, daß man eine Verbindung der Formel I nach Anspruch 1 und/oder eines ihrer physiologisch unbedenklichen Säureadditionssalze zusammen mit mindestens einem festen, flüssigen oder halbflüssigen Träger- und Hilfsstoff in eine geeignete Dosierungsform bringt.

**5.** Pharmazeutische Zubereitung, gekennzeichnet durch einen Gehalt an mindestens einer Verbindung der Formel I nach Anspruch 1 und/oder einem ihrer physiologisch unbedenklichen Säureadditionssalze.

**6.** Verbindung der Formel I nach Anspruch 1 und ihre physiologisch unbedenklichen Säureadditionssalze zur Bekämpfung von Krankheiten.

**7.** Verwendung von Verbindungen der Formel I nach Anspruch 1 und/oder ihrer physiologisch unbedenklichen Säureadditionssalze zur Herstellung eines Arzneimittels.

**8.** Verwendung von Verbindungen der Formel I nach Anspruch 1 und/oder ihrer physiologisch unbedenklichen Säureadditionssalze bei der Bekämpfung von Krankheiten.